# FASCICULE DE BREVET EUROPEEN

(11) **EP 0 669 916 B1**
(45) Date de publication et mention de la délivrance du brevet: **22.01.1997**
(21) Numéro de dépôt: 94900862.7
(22) Date de dépôt: 22.11.1993
(51) Int. Cl.: C07D 305/14, A61K 31/335

(54) **NOUVEAU PROCEDE DE PREPARATION DE DERIVES DU TAXANE, NOUVEAUX DERIVES AINSI OBTENUS ET LES COMPOSITIONS QUI LES CONTIENNENT**
VERFAHREN ZUR HERSTELLUNG VON TAXANDERIVATEN, DIE SO ERLANGTEN NEUEN DERIVATE UND DIESE ENTHALTENDE ZUSAMMENSETZUNGEN
NOVEL METHOD FOR PREPARING TAXANE DERIVATIVES, NOVEL DERIVATIVES THUS OBTAINED AND COMPOSITIONS CONTAINING THEM

(30) Priorité: 23.11.1992 FR 9214023
(43) Date de publication de la demande: 06.09.1995
(73) Titulaire: RHONE-POULENC RORER S.A., 92160 Antony (FR)
(72) Inventeur: BOUCHARD, Hervé, F-94200 Ivry-sur-Seine (FR); BOURZAT, Jean-Dominique, F-94300 Vincennes (FR); COMMERCON, Alain, F-94400 Vitry-sur-Seine (FR)
(74) Mandataire: Pilard, Jacques
(86) Numéro de dépôt international: FR9301145
(87) Numéro de publication internationale: WO9412484

(56) Documents cités:
- EP-A- 0 253 738

## Description

La présente invention concerne la préparation de dérivés du taxane de formule générale : 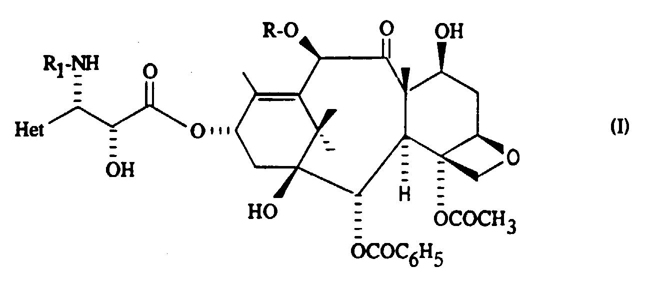 les nouveaux produits ainsi obtenus et les compositions pharmaceutiques qui les contiennent.

Dans la formule générale (I),
R représente un atome d'hydrogène ou un radical acétyle,
R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle, et
Het représente un radical hétérocyclyle aromatique éventuellement substitué ayant 5 chaînons et contenant un ou plusieurs hétéroatomes, identiques ou différents, choisis parmi l'azote, l'oxygène et le soufre.

Plus particulièrement, la présente invention concerne la préparation des produits de formule générale (I) dans laquelle :
R représente un atome d'hydrogène ou un radical acétyle,
R₁ représente un radical benzoyle ou un radical R₂-O-CO dans lequel R₂ représente :
   - un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, nitro, carboxy ou alcoxycarbonyle dont la partie alcoyle contient 1 à 4 atomes de carbone,
   - ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
   - ou un radical hétérocyclyle azoté saturé ou non saturé contenant 5 ou 6 chaînons éventuellement substitué par un ou plusieurs radicaux alcoyles contenant 1 à 4 atomes de carbone,
   étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Het représente un radical hétérocyclique aromatique ayant 5 chaînons et contenant un ou plusieurs atomes, identiques ou différents, choisis parmi les atomes d'azote, d'oxygène ou de soufre, tel que le thiophène, le thiazole, le furanne, le pyrrole, l'imidazole, l'isoxazole ou le pyrazole, éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

D'un intérêt tout particulier est la préparation des produits de formule générale (I) dans laquelle R représente un atome d'hydrogène ou un radical acétyle, R₁ représente un radical benzoyle ou un radical R₂-O-CO- dans lequel R₂ représente un radical t-butyle et Het représente un radical thiényl-2 ou -3 ou furyl-2 ou -3.

Selon la présente invention, les dérivés de formule générale (I) peuvent être obtenus à partir d'un produit de formule générale : 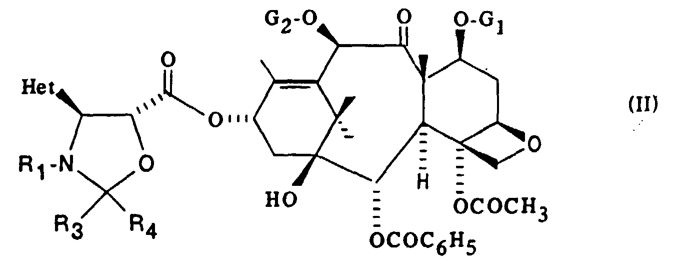 dans laquelle Het et R₁ sont définis comme précédemment, G₁ représente un groupement protecteur de la fonction hydroxy et G₂ représente un radical acétyle ou un groupement protecteur de la fonction hydroxy, et R₃ et R₄, identiques ou différents représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₃ représente un radical alcoxy contenant 1 à 4 atomes de carbone ou un radical trihalométhyle tel que trichlorométhyle ou un radical phényle substitué par un radical trihalométhyle tel que trichlorométhyle et R₄ représente un atome d'hydrogène, ou bien R₃ et R₄ forment ensemble avec l'atome de carbone auquel ils sont liés un cycle ayant 4 à 7 chaînons, en opérant, selon les significations de R₃ et R₄, de la manière suivante :
1) lorsque R₃ représente un atome d'hydrogène ou un radical alcoxy ou un radical aryle éventuellement substitué et R₄ représente un atome d'hydrogène, le produit de formule générale (II) est traité en milieu acide pour obtenir un produit de formule générale : 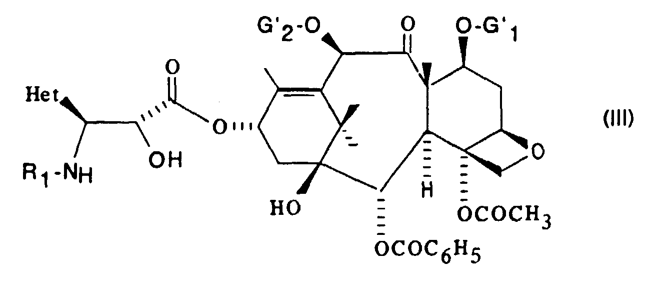 dans laquelle Het et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, dont les groupements protecteurs G'₁ et G'₂ sont, si nécessaire, remplacés par des atomes d'hydrogène pour obtenir un produit de formule générale (I).

La déprotection de la chaîne latérale du produit de formule générale (II) peut être effectuée en présence d'un acide minéral (acide chlorhydrique, acide sulfurique) ou organique (acide acétique, acide méthanesulfonique, acide trifluorométhanesulfonique, acide p-toluènesulfonique), utilisé seul ou en mélange, en opérant dans un solvant organique choisi parmi les alcools (méthanol, éthanol, isopropanol), les éthers (tétrahydrofuranne, éther diisopropylique, méthyl t.butyléther), les esters (acétate d'éthyle, acétate d'isopropyle, acétate de n.butyle), les hydrocarbures aliphatiques (pentane, hexane, heptane), les hydrocarbures aliphatiques halogénés (dichlorométhane, dichloro-1,2 éthane), les hydrocarbures aromatiques (benzène, toluène, xylènes) et les nitriles (acétonitrile) à une température comprise entre - 10 et 60°C, de préférence entre 15 et 30°C. L'acide minéral peut être utilisé en quantité catalytique, stoechiométrique ou en excès.

La déprotection peut aussi être réalisée dans des conditions oxydantes en utilisant par exemple le nitrate d'ammonium et de cérium IV dans un mélange acétonitrile-eau ou la dichloro-2,3 dicyano-5,6 benzoquinone-1,4 dans l'eau.

La déprotection peut être également réalisée dans des conditions réductrices, par exemple par hydrogénolyse en présence d'un catalyseur.

Les radicaux G₁ et G₂, ainsi que G'₁ et G'₂, lorsqu'ils représentent un groupement protecteur de la fonction hydroxy, sont de préférence des radicaux trichloro-2,2,2 éthoxycarbonyle, (trichlorométhyl-2 propoxy)-2 carbonyle ou des radicaux trialkylsilyles, dialkylarylsilyles, alkyldiarylsilyles ou triarylsilyles dans lesquels les parties alkyles contiennent 1 à 4 atomes de carbone et les parties aryles sont de préférence des radicaux phényles.

Lorsque dans la formule générale (II), les groupements protecteurs G₁ et éventuellement G₂ représentent un radical silylé, leur remplacement par des atomes d'hydrogène s'effectue simultanément avec la déprotection de la chaîne latérale.

Le remplacement par des atomes d'hydrogène, dans le produit de formule générale (III), des groupements protecteurs G'₁ et G'₂ représentant un radical trichloro-2,2,2 éthoxycarbonyle ou (trichlorométhyl-2 propoxy)-2 carbonyle est effectué par le zinc, éventuellement associé au cuivre, en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre,
2) lorsque R₃ et R₄, identiques ou différents, représentent un radical alcoyle contenant 1 à 4 atomes de carbone, ou un radical aralcoyle dont la partie alcoyle contient 1 à 4 atomes de carbone et la partie aryle est, de préférence un radical phényle éventuellement substitué, ou un radical aryle, de préférence un radical phényle éventuellement substitué, ou bien R₃ représente un radical trihalométhyle ou un radical phényl substitué par un radical trihalométhyle et R₄ représente un atome d'hydrogène, ou bien R₃ et R₄ forment ensemble avec l'atome de carbone auquel ils sont liés un cycle ayant 4 à 7 chaînons, le produit de formule générale (II) est transformé en produit de formule générale : 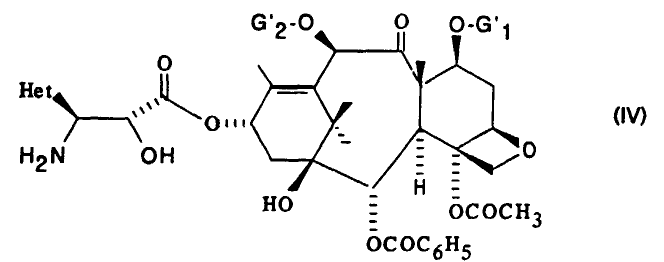 dans laquelle Het est défini comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, qui est acylé au moyen de chlorure de benzoyle ou d'un dérivé réactif de formule générale :

R₂-O-CO-X (V)

dans laquelle R₂ est défini comme précédemment et X représente un atome d'halogène (fluor, chlore) ou un reste -O-R₂ ou -O-CO-O-R₂, pour obtenir un produit de formule générale (III) dont les groupements protecteurs G'₁ et éventuellement G'₂ sont remplacés, si nécessaire, par des atomes d'hydrogène pour obtenir un produit de formule générale (I).

Les produits de formule générale (IV), dans laquelle G'₁ représente un groupement protecteur de la fonction hydroxy choisi parmi les radicaux trichloro-2,2,2 éthoxycarbonyle et (trichlorométhyl-2 propoxy)-2 carbonyle et G'₂ représente un radical acétyle ou un groupement protecteur de la fonction hydroxy choisi parmi les radicaux trichloro-2,2,2 éthoxycarbonyle et (trichlorométhyle-2 propoxy)-2 carbonyle, peuvent être obtenus en traitant un produit de formule générale (II), dans laquelle Het, R₁, G₁ et G₂ sont définis comme ci-dessus, R₃ et R₄, identiques ou différents, représentent un radical alcoyle, aralcoyle ou aryle, ou bien R₃ et R₄ forment ensemble avec l'atome de carbone auquel ils sont liés un cycle ayant 4 à 7 chaînons, par un acide minéral (acide chlorhydrique, acide sulfurique) ou organique (acide formique) éventuellement dans un alcool contenant 1 à 3 atomes de carbone (méthanol; éthanol, isopropanol) à une température comprise entre 0 et 50°C. De préférence, on utilise l'acide formique à une température voisine de 20°C.

Les produits de formule générale (IV), dans laquelle G'₁ représente un atome d'hydrogène et G'₂ représente un radical acétyle peuvent être obtenus en traitant un produit de formule générale (II), dans laquelle G₁ représente un radical silylé et G₂ représente un radical acétyle, R₃ et R₄, identiques ou différents, représentent un radical alcoyle, aralcoyle ou aryle, ou bien R₃ et R₄ forment ensemble avec l'atome de carbone auquel ils sont liés un cycle ayant 4 *à* 7 chaînons, par un acide minéral (acide chlorhydrique, acide sulfurique, acide fluorhydrique) ou organique (acide formique, acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C.

Les produits de formule générale (IV) dans laquelle G'₁ représente un atome d'hydrogène et G'₂ représente un atome d'hydrogène ou un radical acétyle peuvent être obtenus en traitant un produit de formule générale (II), dans laquelle G₁ représente un groupement protecteur choisi parmi les radicaux trichloro-2,2,2 éthoxycarbonyle ou (trichlorométhyl-2 propoxy)-2 carbonyle, G₂ représente un radical acétyle ou un groupement protecteur choisi parmi les radicaux trichloro-2,2,2 éthoxycarbonyle ou (trichlorométhyl-2 propoxy)-2 carbonyle, R₃ représente un radical trihalométhyle ou phényle substitué par un radical trihalométhyle et R₄ représente un atome d'hydrogène, par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone (méthanol, éthanol, propanol, isopropanol) ou dans un ester aliphatique (acétate d'éthyle, acétate d'isopropyle ou acétate de n.butyle) en présence de zinc éventuellement associé à du cuivre.

L'acylation du produit de formule générale (IV) au moyen de chlorure de benzoyle ou d'un dérivé réactif de formule générale (V) est effectuée dans un solvant organique inerte choisi parmi les esters tels que l'acétate d'éthyle, l'acétate d'sopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.

Le remplacement éventuel par des atomes d'hydrogène des groupements protecteurs G'₁ et G'₂ du produit de formule générale (III), lorsqu'ils représentent un radical trichloro-2,2,2 éthoxycarbonyle ou (trichlorométhyl-2 propoxy)-2 carbonyle, est effectué généralement par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone (méthanol, éthanol, isopropanol) ou dans un ester aliphatique (acétate d'éthyle, acétate d'isopropyle, acétate de n.butyle) en présence de zinc éventuellement associé à du cuivre.

Les produits de formule générale (II) peuvent être obtenus par estérification de la baccatine III ou de la désacétyl-10 baccatine III protégée de formule générale : 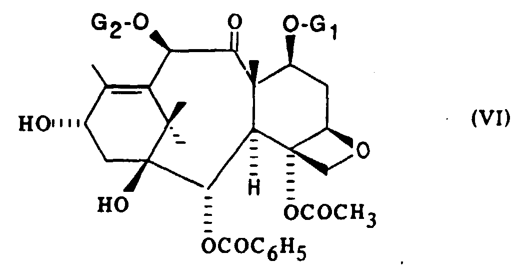 dans laquelle G₁ et G₂ sont définis comme précédemment au moyen d'un acide de formule générale: 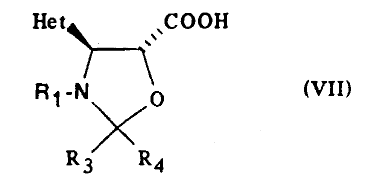 dans laquelle Het, R₁, R₃ et R₄ sont définis comme précédemment, ou d'un dérivé de cet acide.

L'estérification au moyen d'un acide de formule générale (VII) peut être effectuée en présence d'un agent de condensation (carbodiimide, carbonate réactif) et d'un agent d'activation (aminopyridine) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (VII) sous forme d'anhydride en opérant en présence d'un agent d'activation (aminopyridine) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (VII) sous forme d'halogénure ou sous forme d'anhydride avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

L'acide de formule générale (VII) peut être obtenu par saponification d'un ester de formule générale : 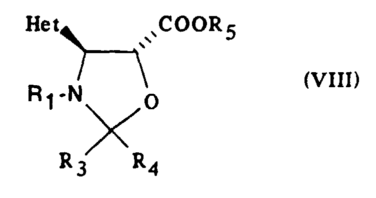 dans laquelle Het, R₁, R₃ et R₄ sont définis comme précédemment et R₅ représente un radical alcoyle contenant 1 à 4 atomes de carbone éventuellement substitué par un radical phényle.

Généralement, la saponification est effectuée au moyen d'une base minérale (hydroxyde, carbonate ou bicarbonate de métal alcalin) en milieu hydro-alcoolique (méthanol-eau) à une température comprise entre 10 et 40°C.

L'ester de formule générale (VIII) peut être obtenu par action d'un produit de formule générale : 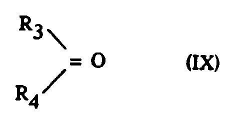 dans laquelle R₃ et R₄ sont définis comme précédemment sous forme d'un dialkylacétal ou d'un alkyléther d'énol, sur un ester de formule générale : 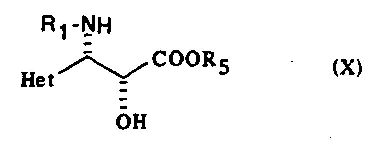 dans laquelle Het, R₁ et R₅ sont définis comme précédemment en opérant dans un solvant organique inerte (hydrocarbure aromatique) en présence d'un acide fort minéral (acide sulfurique) ou organique (acide p.toluènesulfonique éventuellement sous forme de sel de pyridinium) à une température comprise entre 0°C et la température d'ébullition du mélange réactionnel.

L'ester de formule générale (X) peut être obtenu par action d'un produit de formule générale (V) sur un ester de formule générale : 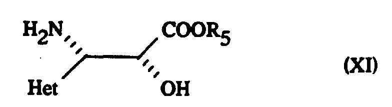 dans laquelle Het et R₅ sont définis comme précédemment, en opérant dans un solvant organique (ester, hydrocarbure aliphatique halogéné) en présence d'une base minérale ou organique à une température comprise entre 0 et 50°C.

Le produit de formule générale (XI) dans laquelle, de préférence, Het représente un hétérocycle soufré, peut être obtenu par réduction d'un azoture de formule générale : 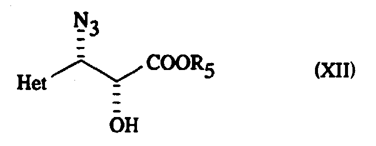 dans laquelle Het et R₅ sont définis comme précédemment, au moyen d'hydrogène en présence d'un catalyseur tel que le palladium sur noir en opérant dans un solvant organique (ester).

Le produit de formule générale (XII) peut être obtenu par action d'un azoture tel que l'azoture de triméthylsilyle en présence de chlorure de zinc ou azoture de métal alcalin (sodium, potassium, lithium) en milieu hydro-organique (eau-tétrahydrofuranne) à une température comprise entre 20°C et la température d'ébullition du mélange réactionnel sur un époxyde de formule générale : 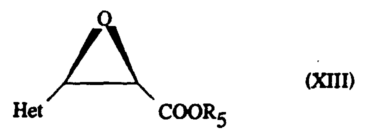 dans laquelle Het et R₅ sont définis comme précédemment, éventuellement préparé in situ.

L'époxyde de formule générale (XIII) peut être obtenu, éventuellement in situ, par déhydrohalogénation d'un produit de formule générale : 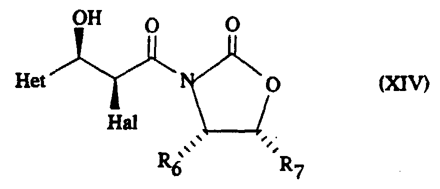 dans laquelle Het est défini comme précédemment, Hal représente un atome d'halogène, de préférence un atome de brome, et R₆ et R₇, identiques ou différents, représentent un atome d'hydrogène ou un radical alcoyle contenant 1 à 4 atomes de carbone ou un radical phényle, l'un au moins étant un radical alcoyle ou un radical phényle, au moyen d'un alcoolate alcalin, éventuellement préparé in situ, dans un solvant organique inerte tel que le tétrahydrofuranne à une température comprise entre -80°C et 25°C.

Le produit de formule générale (XIV) peut être obtenu par action d'un aldéhyde de formule générale:

Het-CHO (XV)

dans laquelle Het est défini comme précédemment sur un halogénure de formule générale : 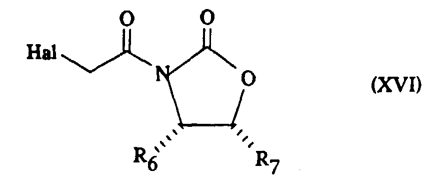 dans laquelle Hal, R₆ et R₇ sont définis comme précédemment, préalablement anionisé.

Généralement, on opère dans un solvant organique inerte choisi parmi les éthers (éther éthylique) et les hydrocarbures aliphatiques halogénés (chlorure de méthylène) à une température comprise entre -80 et 25°C, en présence d'une amine tertiaire (triéthylamine) et d'un agent d'énolisation (triflate de di-n.butylbore).

Le produit de formule générale (XVI) peut être obtenu par action d'un halogénure d'un acide halogénoacétique, de préférence le bromure de l'acide bromoacétique, sur l'oxazolidinone correspondante.

Le produit de formule générale (XI) dans laquelle, de préférence, Het représente un hétérocycle oxygéné, peut être obtenu par hydrogénolyse d'un produit de formule générale : 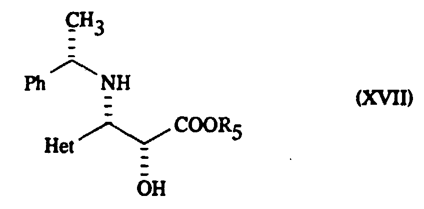 dans laquelle Het et R₅ sont définis comme précédemment et Ph représente un radical phényle éventuellement substitué.

Généralement, l'hydrogénolyse est effectuée au moyen d'hydrogène en présence de catalyseur. Plus particulièrement, on utilise comme catalyseur un palladium sur charbon contenant 1 à 10 % en poids de palladium ou le dihydroxyde de palladium à 20 % en poids de palladium.

L'hydrogénolyse est effectuée dans un solvant organique ou dans un mélange de solvants organiques. Il est avantageux d'opérer dans l'acide acétique éventuellement associé à un alcool aliphatique contenant 1 à 4 atomes de carbone tel qu'un mélange acide acétique-méthanol à une température comprise entre 20 et 80°C.

L'hydrogène nécessaire à l'hydrogénolyse peut aussi être fourni par un composé qui libère de l'hydrogène par réaction chimique ou par décomposition thermique (fonniate d'ammonium). Il est avantageux d'opérer sous une pression d'hydrogène comprise entre 1 et 50 bars.

Le produit de formule générale (XVII) peut être obtenu par hydrolyse ou alcoolyse d'un produit de formule générale : 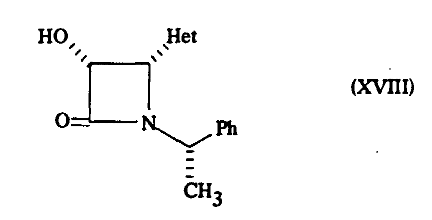 dans laquelle Het et Ph sont définis comme précédemment. Il est particulièrement avantageux d'effectuer une alcoolyse au moyen d'un alcool de formule R₅-OH dans laquelle R₅ est défini comme précédemment en opérant en milieu acide.

De préférence, on effectue l'alcoolyse au moyen de méthanol en présence d'un acide minéral fort tel que l'acide chlorhydrique à une température voisine de la température de reflux du mélange réactionnel.

Le produit de formule générale (XVIII) peut être obtenu par saponification d'un ester de formule générale: 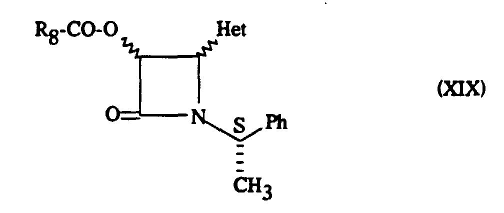 dans laquelle Het et Ph sont définis comme précédemment et R₈ représente un radical alcoyle, phénylalcoyle ou phényle, suivie de la séparation du diastéréoisomère 3R,4S de formule générale (XVIII) des autres diastéréoisomères.

Généralement, la saponification est effectuée au moyen d'une base minérale ou organique telle que l'ammoniaque, la lithine, la soude ou la potasse dans un solvant convenable tel qu'un mélange méthanol-eau ou tétrahydrofuranne-eau à une température comprise entre -10°C et 20°C.

La séparation du diastéréoisomère 3R,4S peut être effectuée par cristallisation sélective dans un solvant organique convenable tel. que l'acétate d'éthyle.

Le produit de formule générale (XIX) peut être obtenu par cycloaddition d'une imine de formule générale : 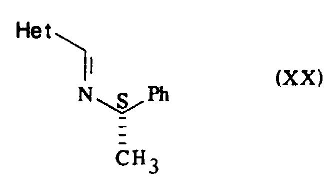 dans laquelle Het et Ph sont définis comme précédemment, sur un halogénure d'acide de formule générale : 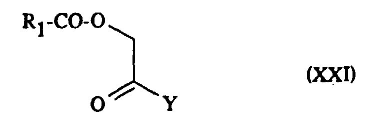 dans laquelle R₁ est défini comme précédemment et Y représente un atome d'halogène tel qu'un atome de brome ou de chlore.

Généralement la réaction est effectuée à une température comprise entre 0 et 50°C en présence d'une base choisie parmi les amines tertiaires aliphatiques (triéthylamine) ou la pyridine dans un solvant organique choisi parmi les hydrocarbures aliphatiques éventuellement halogénés (chlorure de méthylène, chloroforme) et les hydocarbures aromatiques (benzène, toluène, xylènes).

Le produit de formule générale (XX) peut être obtenu dans les conditions analogues à celles décrites par M. Furukawa et coll., Chem. Pharm. Bull., 25 (1), 181-184 (1977).

La baccatine III ou la désacétyl-10 baccatine III protégée de formule générale (VI) peut être obtenue dans les conditions décrites dans les brevets européens EP-0 336 840 et EP-0 336 841.

Les dérivés de formule générale (I) peuvent aussi être obtenus par estérification de la baccatine III ou de la désacétyl-10 baccatine m protégée de formule générale (VI) au moyen d'un acide de formule générale : 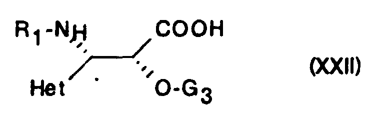 dans laquelle Het et R₁ sont définis comme précédemment et G₃ représente un groupement protecteur de la fonction hydroxy choisi parmi les radicaux méthoxyméthyle, éthoxy-1 éthyle, benzyloxyméthyle, (β-triméthylsilyloxy)méthyle, tétrahydropyranyle, trichloro-2,2,2 éthoxyméthyle, trichloro-2,2,2 éthoxycarbonyle ou CH₂-Ph dans lequel Ph représente un radical phényle éventuellement substitué par un ou plusieurs atomes ou radicaux, identiques ou différents choisis parmi les atomes d'halogène et les radicaux alcoyles contenant 1 à 4 atomes de carbone ou alcoxy contenant 1 à 4 atomes de carbone, ou d'un dérivé activé de cet acide, pour obtenir un produit de formule générale : 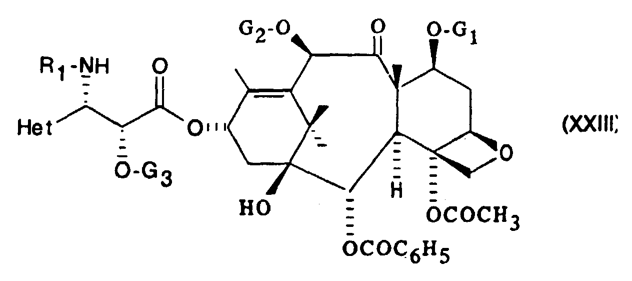 dans laquelle Het, R₁, G₁, G₂ et G₃ sont définis comme précédemment, suivie du remplacement des groupements protecteurs G₁, G₂ et G₃ par des atomes d'hydrogène pour obtenir un produit de formule générale (I).

L'estérification peut être réalisée dans les conditions décrites précédemment pour l'estérification de la baccatine III ou de la désacétyl-10 baccatine III protégée de formule générale (VI) au moyen d'un acide de formule générale (VII).

Le remplacement des groupements protecteurs G₁, G₂ et G₃ du produit de formule générale (XXIII) par des atomes d'hydrogène est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre, lorsque G₁, G₂ et/ou G₃ représentent un radical trichloro-2,2,2 éthoxycarbonyle ou (trichlorométhyl-2 propoxy)-2 carbonyle, ou par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhydrique aqueux à une température comprise entre 0 et 40°C lorsque G₁, G₂ et/ou G₃ représentent un radical silylé. Lorsque G₃ représente un groupement -CH₂-Ph, il est nécessaire de remplacer ce groupement protecteur par un atome d'hydrogène par hydrogénolyse en présence d'un catalyseur, après avoir remplacé les groupements protecteurs G₁ et G₂ par des atomes d'hydrogène dans les conditions décrites précédemment.

L'acide de formule générale (XXII) peut être obtenu par saponification d'un ester de formule générale : 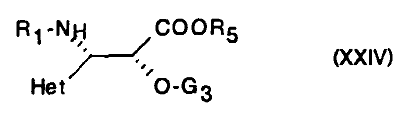 dans laquelle Het, R₁, R₅ et G₃ sont définis comme précédemment.

Généralement la saponification est effectuée au moyen d'une base minérale (hydroxyde, carbonate ou bicarbonate de métal alcalin) en milieu hydro-alcoolique (méthanol-eau) à une température comprise entre 10 et 40°C.

L'ester de formule générale (XXIV) peut être obtenu selon les méthodes habituelles de préparation des éthers, et plus particulièrement selon les procédés décrits par J-N. DENIS et coll., J. Org. Chem., 51, 46-50 (1986), à partir d'un produit de formule générale (X).

Les produits de formule générale (I) obtenus par la mise en oeuvre des procédés selon l'invention peuvent être purifiés selon les méthodes connues telles que la cristallisation ou la chromatographie.

La présente invention concerne également de nouveaux dérivés du taxane de formule génerale : 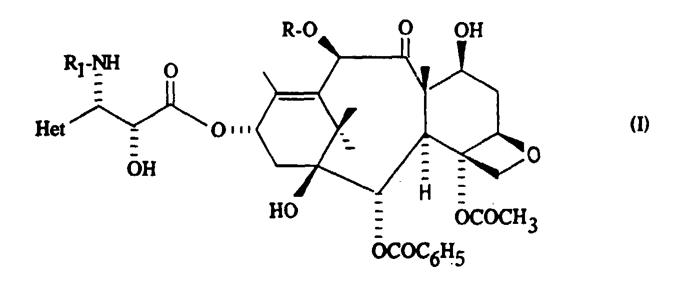 et les compositions pharmaceutiques qui les contiennent.

Dans la formule générale (I),
R représente un atome d'hydrogène ou un radical acétyle,
R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle, et
Het représente un radical hétérocyclyle aromatique éventuellement substitué ayant 5 chaînons et contenant un ou plusieurs hétéroatomes, identiques ou différents, choisis parmi l'azote, l'oxygène et le soufre à l'exception pour Het de représenter un radical furyl-2 ou thiényl-2 lorsque R₁ représente un radical benzoyle ou lorsque R₂ représente un radical alcoyle contenant de 1 à 6 atomes de carbone, les produits correspondants étant décrits dans EP-A-0 534 708.

Dans le brevet européen EP-A-0 253 738 et la demande internationale PCT WO 92/09589 ont été décrits des produits de formule générale (I) dans laquelle le radical hétérocyclique Het est remplacé par un radical phényle éventuellement substitué. Les produits de formule générale (I) selon l'invention ont une activité égale ou supérieure à celle des produits connus antérieurement.

Plus particulièrement, la présente invention concerne les produits de formule générale (I) dans laquelle :
R représente un atome d'hydrogène ou un radical acétyle,
R₁ représente un radical benzoyle ou un radical R₂-O-CO dans lequel R₂ représente :
   - un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, nitro, carboxy ou alcoxycarbonyle dont la partie alcoyle contient 1 à 4 atomes de carbone,
   - ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
   - ou un radical hétérocyclyle azoté saturé ou non saturé contenant 5 ou 6 chaînons éventuellement substitué par un ou plusieurs radicaux alcoyles contenant 1 à 4 atomes de carbone,
   étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Het représente un radical hétérocyclique aromatique ayant 5 chaînons et contenant un ou plusieurs atomes, identiques ou différents, choisis parmi les atomes d'azote, d'oxygène ou de soufre, tel que le thiophène, le thiazole, le furanne, le pyrrole, l'imidazole, l'isoxazole ou le pyrazole, éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbo-nylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone, à l'exception pour Het de représenter un radical furyle-2 ou thiényle-2 lorsque R₁ représente un radical benzoyle ou lorsque R₂ représente un radical alcoyle contenant 1 à 6 atomes de carbone.

D'un intérêt tout particulier sont les produits de formule générale (I) dans laquelle R représente un atome d'hydrogène ou un radical acétyle, R₁ représente un radical benzoyle ou un radical R₂-O-CO- dans lequel R₂ représente un radical t.butyle et Het représente un radical thiényl-3 ou furyl-3.

Les nouveaux produits de formule générale (I) présentent des propriétés biologiques.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) se sont montrés au moins aussi actifs que le taxol et le Taxotère.

In vivo, les produits de formule générale (I) se sont montrés actifs chez la souris greffée par le mélanome B16 à des doses comprises entre 1 et 10 mg/kg par voie intrapéritonéale, ainsi que sur d'autres tumeurs liquides ou solides.

Les exemples suivants illustrent la présente invention.

### EXEMPLE 1

A une solution de 0,67 g d'amino-3 hydroxy-2 (thiényl-3)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans 10 cm3 de dichlorométhane, maintenue sous atmosphère d'argon, on ajoute 0,077 g d'hydrogénocarbonate de sodium puis goutte à goutte, à une température voisine de 20°C, une solution de 0,219 g de dicarbonate de di.tert-butyle dans 10 cm3 de dichlorométhane. La solution obtenue est agitée pendant 20 heures à une température voisine de 20°C puis additionnée d'un mélange de 25 cm3 d'eau distillée et de 20 cm3 de dichlorométhane. La phase aqueuse est séparée par décantation puis extraite par 20 cm3 de dichlorométhane. Les phases organiques sont réunies, séchées sur sulfate de magnésium, filtrées puis concentrées à sec sous pression réduite (2,7 kPa) à 40°C. On obtient 0,76 g d'une meringue blanche que l'on purifie par chromatographie sur 40 g de silice (0,063-0,2 mm) contenus dans une colonne de 2 cm de diamètre [éluant : dichlorométhane-méthanol (99-1 en volumes)] en recueillant des fractions de 3 cm3. Les fractions ne contenant que le produit cherché sont réunies et concentrées à sec sous pression réduite (2,7 kPa) à une température voisine de 40°C. On obtient ainsi 0,564 g de tert-butoxycarbonylamino-3 (thiényl-3)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Une solution de 0,564 g de tert-butoxycarbonylamino-3 (thiényl-3)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans un mélange de 10 cm3 de méthanol et de 10 cm3 d'acide acétique est chauffée sous agitation et sous atmosphère d'argon jusqu'à une température voisine de 60°C puis additionnée de 1,2 g de zinc en poudre. Le mélange réactionnel est ensuite agité pendant 15 minutes à 60°C puis refroidi à une température voisine de 20°C et filtré sur verre fritté garni de célite. Le verre fritté est lavé par 3 fois 10 cm3 de dichlorométhane et les filtrats sont réunis puis concentrés à sec sous pression réduite (2,7 kPa) à une température voisine de 40°C.

Le résidu est additionné de 25 cm3 d'eau distillée et le solide cristallisé est séparé par filtration, lavé par 4 fois 5 cm3 d'eau distillée et séché sous pression réduite (0,27 kPa) à 20°C pendant 16 heures. On obtient 0,30 g d'une meringue blanche que l'on purifie par chromatographie sur 40 g de silice (0,063-0,2 mm) contenus dans une colonne de 2 cm de diamètre [éluant : dichlorométhane-méthanol (97-3 en volumes)] en recueillant des fractions de 5 cm3. Les fractions ne contenant que le produit cherché sont réunies et concentrées à sec sous pression réduite (0,27 kPa) à 40°C pendant 16 heures. On obtient ainsi 0,18 g de tert-butoxycarbonylamino-3 (thiényl-3)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D} = -30° (c = 0,36 ; méthanol)
- spectre de RMN (400 MHz ; CDCl₃ ; δ en ppm) : 1,20 (s, 3H : -CH₃ 16 ou 17) ; 1,30 (s, 3H : -CH₃ 16 ou 17) ; 1,40 (s, 9H : -C(CH₃)₃) ; 1,70 (s, 1H : -OH 1) ; 1,82 (s, 3H : -CH₃ 19) ; 1,88 (m,1H:-(CH)-H 6) ; 1,95 (s, 3H : -CH₃ 18) ; 2,40 (m, 5H : -CH₂- 14 et -COCH₃) ; 2,62 [m, 1H : -(CH)-H 6] ; 3,40 (d, 1H : -OH 2') ; 4,00 (d, 1H : -H 3) ; 4,20 (bs, 1H : -OH 10) ; 4,25 [m, 2H : -H 7 et -(CH)-H 20] ; 4,35 [d, 1H: -(CH)-H 20] ; 4,65 (dd, 1H : -H 2') ; 4,97 (dd, 1H : -H 5) ; 5,20 à 5,40 [m, 3H : -H 3', -H 10 et -NHCOOC(CH₃)₃] ; 5,75 (d, 1H : -H 2) ; 6,25 (t, 1H : -H 13) ; 7,15 [d, 1H : (-H 4)-thiényl-3] ; 7,35 [bs, 1H : (-H 2)-thiényl-3] ; 7,40 [dd, 1H : (-H 5)-thiényl-3] ; 7,55 [dd, 2H : -OCOC₆H₅(-H 3 et -H 5)] ; 7,65 [t, 1H : -OCOC₆H₅ (-H 4)] ; 8,15 [d, 2H : -OCOC₆H₅(-H 2 et -H 6)].

L'amino-3 hydroxy-2 (thiényl-3)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

Une solution de 0,87 g de tert-butoxycarbonyl-3 (méthoxy-4 phényl)-2 (thiényl-3)-4 oxazolidinecarboxylate-5-(2RS ,4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans 8 cm3 d'acide formique est agitée pendant 4 heures à une température voisine de 20°C puis concentrée à sec sous pression réduite (2,7 kPa) à 40°C. Le résidu est additionné d'un mélange de 100 cm3 de dichlorométhane et de 15 cm3 d'une solution aqueuse saturée d'hydrogénocarbonate de sodium. La phase aqueuse est séparée par décantation et extraite par 15 cm3 de dichlorométhane. Les phases organiques sont réunies, séchées sur sulfate de magnésium, filtrées puis concentrées à sec sous pression réduite (2,7 kPa) à 40°C. On obtient 0,73 g d'une meringue blanche que l'on purifie par chromatographie sur 40 g de silice (0,063-0,2 mm) contenus dans une colonne de 2 cm de diamètre [éluant : dichlorométhane-méthanol (98-2 en volumes)] en recueillant des fractions de 5 cm3. Les fractions ne contenant que le produit cherché sont réunies et concentrées à sec sous pression réduite (2,7 kPa) à une température voisine de 40°C. On obtient ainsi 0,455 g d'amino-3 hydroxy-2 (thiényl-3)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Le tert-butoxycarbonyl-3 (méthoxy-4 phényl)-2 (thiényl-3)-4 oxazolidine-carboxylate-5-(2RS,4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

Une solution de 0,45 g d'acide tert-butoxycarbonyl-3 (méthoxy-4 phényl)-2 (thiényl-3)-4 oxazolidinecarboxylique-5-(2RS,4S,5R) et de 0,602 g d'acétoxy4 benzoyloxy-2α époxy-5β,20 dihydroxy-1,13α oxo-9 bis-(trichloro-2,2,2 éthoxy)carbonyloxy-7β,10β taxène-11 dans 100 cm3 de toluène est déshydratée par distillation azéotropique du toluène à une température voisine de 60°C et sous une pression de 13,3 kPa. On élimine ainsi 10 cm3 de toluène en 20 minutes. Après avoir refroidi le milieu réactionnel à une température voisine de 20°C, on ajoute 0,277 g de N,N'-dicyclohexylcarbodiimide et 0,041 g de diméthylamino-4 pyridine. Le milieu réactionnel est ensuite agité pendant 1 heure 30 minutes à une température voisine de 20°C, puis additionné d'un mélange de 250 cm3 de dichlorométhane et de 7 cm3 d'une solution aqueuse saturée d'hydrogénocarbonate de sodium. La phase aqueuse est séparée par décantation puis extraite par 15 cm3 de dichlorométhane. Les phases organiques sont réunies, séchées sur du sulfate de magnésium, filtrées puis concentrées à sec sous pression réduite (2,7 kPa) à 50°C. On obtient 1,36 g d'une meringue blanche que l'on purifie par chromatographie sur 50 g de silice (0,063-0,2 mm) contenus dans une colonne de 2 cm de diamètre [éluant : dichlorométhane-méthanol (99-1 en volumes)] en recueillant des fractions de 10 cm3. Les fractions ne contenant que le produit cherché sont réunies et concentrées à sec sous pression réduite (2,7 kPa) à une température voisine de 40°C. On obtient ainsi 0,83 g de tert-butoxycarbonyl-3 (méthoxy-4 phényl)-2 (thiényl-3)-4 oxazolidinecarboxylate-5-(2RS,4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'acétoxy-4 benzoyloxy-2α époxy-5β,20 dihydroxy-1,13α oxo-9 bis-(trichloro-2,2,2 éthoxy)carbonyloxy-7β,10β taxène-11 peut être préparé selon la méthode décrite dans le brevet européen EP-0 336 841.

L'acide tert-butoxycarbonyl-3 (méthoxy-4 phényl)-2 (thiényl-3)-4 oxazolidinecarboxylique-5-(2RS,4S,5R) peut être préparé de la manière suivante :

A une solution de 1,36 g de tert-butoxycarbonyl-3 (méthoxy-4 phényl)-2 (thiényl-3)-4 oxazolidinecarboxylate-5-(2RS,4S,5R) d'éthyle dans 20 cm3 d'éthanol, on ajoute à une température voisine de 25°C, une solution de 0,4 g d'hydrate d'hydroxyde de lithium dans 1,5 cm3 d'eau distillée. Le milieu réactionnel est agité pendant 1 heure à une température voisine de 25°C puis concentré à sec sous pression réduite (2,7 kPa) à une température voisine de 40°C. Le résidu obtenu est dissous dans 50 cm3 d'eau distillée puis extrait par 3 fois 50 cm3 d'éther diéthylique. La phase aqueuse est ensuite acidifiée à un pH voisin de 1 par une solution aqueuse 1N d'acide chlorhydrique, puis extraite par 3 fois 50 cm3 de dichlorométhane.

Les phases organiques sont réunies, séchées sur sulfate de magnésium, filtrées puis concentrées à sec sous pression réduite (2,7 kPa) à une température voisine de 40°C. On obtient ainsi 1 g d'acide tert-butoxycarbonyl-3 (méthoxy-4 phényl)-2 (thiényl-3)-4 oxazolidinecarboxylique-5-(2RS,4S,5R) sous forme d'une meringue blanche.

Le tert-butoxycarbonyl-3 (méthoxy-4 phényl)-2 (thiényl-3)-4 oxazolidine-carboxylate-5-(2RS,4S,5R) d'éthyle peut être préparé de la manière suivante :

Une solution de 1,9 g de tert-butoxycarbonylamino-3 hydroxy-2 (thiényl-3)-3 propionate-(2R,3S) d'éthyle et de 75 mg de p-toluène sulfonate de pyridinium dans 18 cm3 de toluène est chauffé jusqu'à l'ébullition et le distillat est recueilli dans un appareil de Dean-Stark. Après avoir éliminé 10 cm3 de distillat on ajoute, goutte à goutte, une solution de 1,06 cm3 de l'acétal diméthylique de l'anisaldéhyde dans 6 cm3 de toluène et maintient le reflux pendant 2 heures 30 minutes. Le milieu réactionnel est refroidi à une température voisine de 40°C et concentré à sec sous pression réduite (2,7 kPa) à une température voisine de 40°C. On obtient 2,62 g d'une huile brune que l'on purifie par chromatographie sur 100 g de silice (0,04-0,063 mm) contenus dans une colonne de 5 cm de diamètre [éluant : éther de pétrole-éther diéthylique (80-20 en volumes)] en recueillant des fractions de 4 cm3. Les fractions ne contenant que le produit cherché sont réunies et concentrées à sec sous pression réduite (2,7 kPa) à une température voisine de 40°C. On obtient ainsi 1,8 g de tert-butoxycarbonyl-3 (méthoxy-4 phényl)-2 (thiényl-3)-4 oxazolidinecarboxylate-5-(2RS,4S,5R) d'éthyle sous forme d'une huile jaune.

Le tert-butoxycarbonylamino-3 hydroxy-2 (thiényl-3)-3 propionate-(2R,3S) d'éthyle peut être préparé de la manière suivante :

A une solution de 3,75 g d'amino-3 hydroxy-2 (thiényl-3)-3 propionate-(2R,3S) d'éthyle dans 100 cm3 de dichlorométhane, maintenue sous atmosphère d'argon, on ajoute 1,65 g d'hydrogénocarbonate de sodium puis goutte à goutte, à une température voisine de 20°C, une solution de 4,7 g de dicarbonate de di.tert-butyle dans 10 cm3 de dichlorométhane. La solution obtenue est agitée pendant 72 heures à une température voisine de 20°C puis additionnée de 60 cm3 d'eau distillée. La phase aqueuse est séparée par décantation puis extraite par 3 fois 50 cm3 de dichlorométhane. Les phases organiques sont réunies, séchées sur sulfate de magnésium, filtrées puis concentrées à sec sous pression réduite (2,7 kPa) à 40°C. On obtient 9,15 g d'une huile brune que l'on purifie par chromatographie sur 300 g de silice (0,063-0,2 mm) contenus dans une colonne de 5 cm de diamètre [éluant : cyclohexane-acétate d'éthyle (70-30 en volumes)] en recueillant des fractions de 20 cm3. Les fractions ne contenant que le produit cherché sont réunies et concentrées à sec sous pression réduite (2,7 kPa) à une température voisine de 40°C. On obtient ainsi 4,95 g de tert-butoxycarbonylamino-3 hydroxy-2 (thiényl-3)-3 propionate-(2R,3S) d'éthyle sous forme d'une huile incolore qui cristallise à température ambiante.

L'amino-3 hydroxy-2 (thiényl-3)-3 propionate-(2R,3S) d'éthyle peut être préparé de la manière suivante :

A une solution de 4,3 g d'azido-3 hydroxy-2 (thiényl-3)-3 propionate-(2R,3S) d'éthyle dans 70 cm3 d'acétate d'éthyle on ajoute 0,2 g de palladium à 10 % sur poudre de carbone. Le mélange réactionnel est agité sous une pression de 120 kPa d'hydrogène et à une température voisine de 22°C pendant 21 heures puis filtré sur verre fritté garni de célite. Le verre fritté est lavé par 3 fois 5 cm3 d'acétate d'éthyle et les filtrats sont réunis puis concentrés à sec sous pression réduite (2,7 kPa) à une température voisine de 40°C. On obtient ainsi 3,75 g d'amino-3 hydroxy-2 (thiényl 3)-3 propionate-(2R,3S) d'éthyle sous forme de cristaux blancs fondant à 85°C.

L'azido-3 hydroxy-2 (thiényl-3)-3 propionate-(2R,3S) d'éthyle peut être préparé de la manière suivante :

A une solution, refroidie à une température voisine de -75°C, de 0,9 cm3 d'éthanol dans 15 cm3 de tétrahydrofuranne anhydre on ajoute en maintenant la température à -75°C, 9,6 cm3 d'une solution 1,6M de n-butyllithium dans l'hexane puis, goutte à goutte, une solution de 3,1 g de [bromo-2 hydroxy-3 (thiényl-3)-3 oxo-1 propyl]-3-(2S,3R) méthyl-4 phényl-5 oxazolidinone-2-(4S,5R) dans 50 cm3 de tétrahydrofuranne. Le milieu réactionnel est réchauffé jusqu'à une température voisine de 15°C puis maintenu à 15°C pendant 15 minutes et refroidi à nouveau à une température voisine de -75°C. On ajoute ensuite en maintenant la température à -75°C, une solution de 2,0 g d'acide citrique dans 10 cm3 de tétrahydrofuranne. Le milieu réactionnel est réchauffé jusqu'à une température voisine de -10°C et additionné de 20 cm3 d'eau distillée. La phase organique est décantée et lavée par 2 fois 10 cm3 d'une solution saturée de chlorure de sodium.

A cette phase organique on ajoute successivement 25 cm3 d'éther monométhylique de l'éthylèneglycol, 25 cm3 d'eau distillée, 2,45 g d'azoture de sodium et 1,0 g de chlorure d'ammonium. Le mélange réactionnel est agité à reflux pendant 30 heures puis refroidi à une température voisine de 20°C. Les solvants organiques sont éliminés par concentration sous pression réduite (2,7 kPa) à 40°C.

Le résidu obtenu est additionné de 10 cm3 d'éther diéthylique. La phase aqueuse est séparée par décantation et extraite par 4 fois 10 cm3 d'éther diéthylique. Les phases organiques sont réunies, séchées sur sulfate de magnésium, filtrées puis concentrées à sec sous pression réduite (2,7 kPa) à 40°C. On obtient 2,47 g d'une huile orange que l'on purifie par chromatographie sur 100 g de silice (0,063-0,2 mm) contenus dans une colonne de 2 cm de diamètre [éluant : cyclohexane-acétate d'éthyle (90-10 en volumes)] en recueillant des fractions de 5 cm3. Les fractions ne contenant que le produit cherché sont réunies et concentrées à sec sous pression réduite (2,7 kPa) à une température voisine de 40°C. On obtient ainsi 1,44 g d'azido-3 hydroxy-2 (thiényl-3)-3 propionate-(2R,3S) d'éthyle sous forme d'une huile jaune pâle.

La [bromo-2 hydroxy-3 (thiényl-3)-3 oxo-1 propyl]-3-(2S,3R) méthyl-4 phényl-5 oxazolidinone-2-(4S,5R) peut être préparé de la manière suivante :

A une solution de 19 g de (bromo-2 oxo-1 éthyl)-3 méthyl-4 phényl-5 oxazolidinone-2-(4S,5R) dans 300 cm3 d'éther diéthylique anhydre, on ajoute à une température voisine de 20°C, 12,6 cm3 de triéthylamine puis, goutte à goutte, 70,4 cm3 d'une solution 1M de di-n-butyl triflate de bore dans le dichlorométhane. Le milieu réactionnel est refroidi à une température voisine de -75°C puis on ajoute en maintenant la température à -75°C, une solution de 4,2 cm3 de thiophènecarbaldéhyde-3 dans 10 cm3 d'éther diéthylique et réchauffe le milieu réactionnel jusqu'à une température voisine de 0°C et le maintient à 0°C pendant 1 heure 30 minutes. On ajoute ensuite 40 cm3 d'une solution saturée d'hydrogénosulfate de sodium, sépare la phase aqueuse par décantation et l'extrait par 2 fois 50 cm3 d'éther diéthylique. Les phases organiques sont réunies, séchées sur sulfate de magnésium, filtrées puis concentrées à sec sous pression réduite (2,7 kPa) à 40°C. On obtient 43,2 g d'une huile brune que l'on purifie par chromatographie sur 500 g de silice (0,063-0,2 mm) contenus dans une colonne de 5 cm de diamètre [éluant : cyclohexane-acétate d'éthyle (90-10 en volumes)] en recueillant des fractions de 100cm3. Les fractions ne contenant que le produit cherché sont réunies et concentrées à sec sous pression réduite (2,7 kPa) à une température voisine de 40°C. On obtient ainsi, après cristallisation dans l'oxyde de diisopropyle 14 g de [bromo-2 hydroxy-3 (thiényl-3)-3 oxo-1 propyl]-3-(2S,3R) méthyl-4 phényl-5 oxazolidinone-2-(4S,5R) fondant à 124°C.

La (bromo-2 oxo-1 éthyl)-3 méthyl-4 phényl-5 oxazolidinone-2-(4S,5R) peut être préparée dans les conditions décrites dans la demande internationale PCT WO 92/09589.

### EXEMPLE 2

A une solution de 0,75 g d'amino-3 hydroxy-2 (furyl-3)-3 propionate-(2R,3S) d'acétoxy4 benzoyloxy-2α époxy-5β,20 hydroxy-1 oxo-9 bis-(trichloro-2,2,2 éthoxy)carbonyloxy-7β,10β taxène-11 yle-13α dans 40 cm3 de dichlorométhane, maintenue sous atmosphère d'argon, on ajoute 0,066 g d'hydrogénocarbonate de sodium puis, goutte à goutte, à une température voisine de 20°C, une solution de 0,188 g de dicarbonate de di.tert-butyle dans 10 cm3 de dichlorométhane. La solution obtenue est agitée pendant 20 heures à une température voisine de 20°C puis additionnée d'un mélange de 25 cm3 d'eau distillée et de 20 cm3 de dichlorométhane. La phase aqueuse est séparée par décantation puis extraite par 20 cm3 de dichlorométhane. Les phases organiques sont réunies, séchées sur sulfate de magnésium, filtrées puis concentrées à sec sous pression réduite (2,7 kPa) à 40°C. On obtient 0,85 g d'une meringue blanche que l'on purifie par chromatographie sur 70 g de silice (0,063-0,2 mm) contenus dans une colonne de 2,5 cm de diamètre [éluant : dichlorométhane-méthanol (99-1 en volumes)] en recueillant des fractions de 5 cm3. Les fractions ne contenant que le produit cherché sont réunies et concentrées à sec sous pression réduite (2,7 kPa) à une température voisine de 40°C. On obtient ainsi 0,74 g de tert-butoxycarbonylamino-3 (furyl-3)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Une solution de 0,73 g de tert-butoxycarbonylamino-3 (furyl-3)-3 hydroxy-2 propîonate-(2R,3S) d'acétoxy4 benzoyloxy-2α époxy-5β,20 hydroxy-1 oxo-9 bis-(trichloro-2,2,2 éthoxy)carbonyloxy-7β,10β taxène-11 yle-13α dans un mélange de 15 cm3 de méthanol et de 15 cm3 d'acide acétique est chauffée sous agitation et sous atmosphère d'argon jusqu'à une température voisine de 60°C puis additionnée de 1,5 g de zinc en poudre. Le mélange réactionnel est ensuite agité pendant 10 minutes à 60°C puis refroidi à une température voisine de 20°C et filtré sur verre fritté garni de célite. Le verre fritté est lavé par 3 fois 10 cm3 de méthanol et les filtrats sont réunis puis concentrés à sec sous pression réduite (2,7 kPa) à une température voisine de 40°C.

Le résidu est additionné de 15 cm3 d'eau distillée et le solide cristallisé est séparé par filtration, lavé par 6 fois 5 cm3 d'eau distillée et séché sous pression réduite (0,27 kPa) à 20°C pendant 16 heures. On obtient 0,49 g d'une meringue blanche que l'on purifie par chromatographie sur 40 g de silice (0,063-0,2 mm) contenus dans une colonne de 2 cm de diamètre [éluant : dichlorométhane-méthanol (97-3 en volumes)] en recueillant des fractions de 5 cm3. Les fractions ne contenant que le produit cherché sont réunies et concentrées à sec sous pression réduite (0,27 kPa) à 40°C pendant 16 heures. On obtient ainsi 0,31 g de tert-butoxycarbonylamino-3 (furyl-3)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D} = -33° (c = 0,47 ; méthanol)
- spectre de RMN (400 MHz ; CDCl₃ ; δ en ppm) : 1,20 (s, 3H : -CH₃ 16 ou 17) ; 1,30 (s, 3H : -CH₃ 16 ou 17) ; 1,40 (s, 9H : -C(CH₃)₃) ; 1,72 (s, 1H : -OH 1) ; 1,80 (s, 3H : -CH₃ 19) ; 1,90 [m, 1H : -(CH)-H 6] ; 1,92 (s, 3H : -CH₃ 18) ; 2,35 (m, 5H : -CH₂- 14 et -COCH₃) ; 2,60 [m, 1H : -(CH)-H 6] ; 3,50 (d, 1H : -OH 2') ; 3,95 (d, 1H : -H 3) ; 4,25 [m, 3H : -OH 10, -(CH)-H 20 et -H 7] ; 4,35 [d, 1H : -(CH)-H 20] ; 4,58 (dd, 1H : -H 2') ; 5,00 (dd, 1H : -H 5) ; 5,20 [m, 3H : -H 3', -H 10 et -NHCOOC (CH₃)₃] ; 5,70 (d, 1H : -H 2) ; 6,25 (t, 1H : -H 3) ; 6,50 [bs, 1H : (-H 4)-furyl-3] ; 7,45 [bd, 1H : (-H 2)-furyl-3] ; 7,55 [m, 3H : -OCOC₆H₅(-H 3 et -H 5) et (-H 5)-furyl-3] ; 7,65 [t, 1H : -OCOC₆H₅(-H 4)] ; 8,15 [d, 2H : -OCOC₆H₅(-H 2 et -H 6)].

L'amino-3 hydroxy-2 (furyl-3)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

Une solution de 1,15 g de tert-butoxycarbonyl-3 (méthoxy-4 phényl)-2 (furyl-3)-4 oxazolidinecarboxylate-5-(2RS,4S,5R) d'acétoxy4 benzoyloxy-2α époxy-5β,20 hydroxy-1 oxo-9 bis-(trichloro-2,2,2 éthoxy)carbonyloxy-7β,10β taxène-11 yle-13α dans 20 cm3 d'acide formique est agitée pendant 4 heures à une température voisine de 20°C puis concentrée à sec sous pression réduite (2,7 kPa) à 40°C. Le résidu est additionné d'un mélange de 100 cm3 de dichlorométhane et de 25 cm3 d'une solution aqueuse saturée d'hydrogénocarbonate de sodium. La phase aqueuse est séparée par décantation et extraite par 15 cm3 de dichlorométhane. Les phases organiques sont réunies, séchées sur sulfate de magnésium, filtrées puis concentrées à sec sous pression réduite (2,7 kPa) à 40°C. On obtient 1,5 g d'une meringue blanche que l'on purifie par chromatographie sur 70 g de silice (0,063-0,2 mm) contenus dans une colonne de 2,5 cm de diamètre [éluant : dichlorométhane-méthanol (98-2 en volumes)] en recueillant des fractions de 5 cm3. Les fractions ne contenant que le produit cherché sont réunies et concentrées à sec sous pression réduite (2,7 kPa) à une température voisine de 40°C. On obtient ainsi 0,76 g d'amino-3 hydroxy-2 (furyl-3)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue orangé.

Le tert-butoxycarbonyl-3 (méthoxy-4 phényl)-2 (furyl-3)-4 oxazolidinecarboxylate-5-(2R5,4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

Une solution de 0,66 g d'acide tert-butoxycarbonyl-3 (méthoxy-4 phényl)-2 (furyl-3)-4 oxazolidinecarboxylique-5-(2RS,4S,5R) et de 1,0 g d'acétoxy-4 benzoyloxy-2a époxy-5β,20 dihydroxy-1,13α oxo-9 bis-(trichloro-2,2,2 éthoxy)carbonyloxy-7β,10β taxène-11 dans 50 cm3 de toluène est déshydratée par distillation azéotropique du toluène à une température voisine de 60°C et sous une pression de 13,3 kPa. On élimine ainsi 10 cm3 de toluène en 20 minutes. Après avoir refroidi le milieu réactionnel à une température voisine de 20°C, on ajoute 0,450 g de N,N'-dicyclohexylcarbodiimide et 0,067 g de diméthylamino-4 pyridine. Le milieu réactionnel est ensuite agité pendant 2 heures 30 minutes à une température voisine de 20°C, puis additionné d'un mélange de 250 cm3 de dichlorométhane et de 25 cm3 d'une solution aqueuse saturée d'hydrogénocarbonate de sodium. La phase aqueuse est séparée par décantation puis extraite par 50 cm3 de dichlorométhane. Les phases organiques sont réunies, séchées sur du sulfate de magnésium, filtrées puis concentrées à sec sous pression réduite (2,7 kPa) à 40°C. On obtient 3,0 g d'une meringue blanche que l'on purifie par chromatographie sur 80 g de silice (0,063-0,2 mm) contenus dans une colonne de 2,5 cm de diamètre [éluant : dichlorométhane-méthanol (99,5-0,5 en volumes)] en recueillant des fractions de 10 cm3. Les fractions ne contenant que le produit cherché sont réunies et concentrées à sec sous pression réduite (2,7 kPa) à une température voisine de 40°C. On obtient ainsi 1,15 g de tert-butoxycarbonyl-3 (méthoxy-4 phényl)-2 (furyl-3)-4 oxazolidinecarboxylate-5-(2RS,4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'acide tert-butoxycarbonyl-3 (méthoxy-4 phényl)-2 (furyl-3)-4 oxazolidinecarboxylique-5-(2RS,4S,5R) peut être préparé de la manière suivante :

A une solution de 0,775 g de tert-butoxycarbonyl-3 (méthoxy-4 phényl)-2 (furyl-3)-4 oxazolidinecarboxylate-5-(2RS,4S,5R) de méthyle dans 20 cm3 d'éthanol, on ajoute à une température voisine de 25°C, une solution de 0,24 g d'hydrate d'hydroxyde de lithium dans 7 cm3 d'eau distillée.

Le milieu réactionnel est agité pendant 15 minutes à une température voisine de 25°C puis concentré à sec sous pression réduite (2,7 pKa) à une température voisine de 40°C. Le résidu obtenu est dissous dans 30 cm3 d'eau distillée puis extrait par 3 fois 50 cm3 d'éther diéthylique. La phase aqueuse est ensuite acidifiée à un pH voisin de 3 par une solution aqueuse 2N d'acide chlorhydrique, puis extraite par 3 fois 25 cm3 de dichlorométhane. Les phases organiques sont réunies, séchées sur sulfate de magnésium, filtrées puis concentrées à sec sous pression réduite (2,7 kPa) à une température voisine de 40°C. On obtient ainsi 0,690 g d'acide tert-butoxycarbonyl-3 (méthoxy-4 phényl)-2 (furyl-3)-4 oxazolidinecarboxylique-5-(2RS,4S,5R) sous forme d'une meringue blanche.

Le tert-butoxycarbonyl-3 (méthoxy-4 phényl)-2 (furyl-3)-4 oxazolidinecarboxylate-5-(2RS,4S,5R) de méthyle peut être préparé de la manière suivante :

Une solution de 0,75 g de tert-butoxycarbonylamino-3 hydroxy-2 (furyl-3)-3 propionate-(2R,3S) de méthyle et de 33 mg de p-toluène sulfonate de pyridinium dans 35 cm3 de toluène est chauffé jusqu'à l'ébullition et le distillat est recueilli dans un appareil de Dean-Stark. Après avoir éliminé 10 cm3 de distillat on ajoute, goutte à goutte, une solution de 0,446 cm3 de l'acétal diméthylique de l'anisaldéhyde dans 5 cm3 de toluène et maintient le reflux pendant 1 heure 30 minutes. Le milieu réactionnel est refroidi à une température voisine de 40°C et concentré à sec sous pression réduite (2,7 kPa) à une température voisine de 40°C. On obtient 1,4 g d'une huile brune que l'on purifie par chromatographie sur 70 g de silice (0,063-0,2 mm) contenus dans une colonne de 2,5 cm de diamètre [éluant : dichlorométhane-méthanol (99,5-0,5 en volumes)] en recueillant des fractions de 5 cm3. Les fractions ne contenant que le produit cherché sont réunies et concentrées à sec sous pression réduite (2,7 kPa) à une température voisine de 40°C. On obtient ainsi 0,8 g de tert-butoxycarbonyl-3 (méthoxy-4 phényl)-2 (furyl-3)-4 oxazolidinecarboxylate-5-(2RS,4S,5R) de méthyle sous forme d'une huile jaune pâle.

Le tert-butoxycarbonylamino-3 hydroxy-2 (furyl-3)-3 propionate-(2R,3S) de méthyle peut être préparé de la manière suivante :

A une solution de 0,92 g d'amino-3 hydroxy-2 (furyl-3)-3 propionate-(2R,3S) de méthyle dans 30 cm3 de dichlorométhane, maintenue sous atmosphère d'argon, on ajoute 0,46 g d'hydrogénocarbonate de sodium puis goutte à goutte, à une température vosine de 20°C, une solution de 1,23 g de dicarbonate de di.tert-butyle dans 5 cm3 de dichlorométhane. La solution obtenue est agitée pendant 20 heures à une température voisine de 20°C puis additionnée de 30 cm3 d'eau distillée. La phase aqueuse est séparée par décantation puis extraite par 3 fois 25 cm3 de dichlorométhane. Les phases organiques sont réunies, séchées sur sulfate de magnésium, filtrées puis concentrées à sec sous pression réduite (2,7 kPa) à 40°C. On obtient 1,36 g d'une huile jaune pâle qui cristallise à température ambiante.

L'amino-3 hydroxy-2 furyl-3 propionate-(2R,3S) de méthyle peut être préparé de la manière suivante :

A 3,0 g d'une dispersion à 3 % de palladium dans du charbon activé en poudre on ajoute une solution de 2,42 g d'hydroxy-2 [phényl-1-(S)] éthylamino-3 furyl-3 propionate-(2R,3S) de méthyle dans un mélange de 40 cm3 de méthanol et de 0,48 cm3 d'acide acétique. Le mélange réactionnel est agité pendant 1 heure à 20°C sous une pression de 120 kPa d'hydrogène. Le mélange réactionnel est ensuite filtré sur verre fritté garni de célite. Le verre fritté est lavé par 3 fois 15 cm3 de méthanol et les filtrats sont réunis puis concentrés à sec sous pression réduite (2,7 kPa) à une température voisine de 40°C. Le résidu est additionné de 40 cm3 d'eau distillée et alcalinisé à un pH voisin de 7 par addition d'une solution aqueuse 7,5N d'hydroxyde de sodium puis extrait par 4 fois 100 cm3 de dichlorométhane. Les phases organiques sont réunies, séchées sur sulfate de magnésium, filtrées puis concentrées à sec sous pression réduite (2,7 kPa) à 40°C. L'huile résiduelle brune est purifiée par chromatographie sur 60 g de silice (0,04-0,063 mm) contenus dans une colonne de 2,5 cm de diamètre [éluant : acétate d'éthyle-méthanol (95-5 en volumes)]. Les fractions ne contenant que le produit cherché sont réunies et concentrées à sec sous pression réduite (2,7 kPa) à une température voisine de 40°C. On obtient ainsi 0,93 g d'amino-3 hydroxy-2 furyl-3 propionate-(2R,3S) de méthyle sous forme d'une huile jaune.

L'hydroxy-2 [(phényl-1(S)] éthylamino-3 furyl-3 propionate-(2R,3S) de méthyle peut être préparé de la manière suivante :

Une solution de 2,18 g d'hydroxy-3 (furyl-3)-4 [phényl-1-(S)] éthyl-1 azétidinone-2-(3R,4S) dans un mélange de 40 cm3 de méthanol et de 4 cm3 d'une solution aqueuse 12N d'acide chlorhydrique est chauffée au reflux (65°C) pendant 16 heures, puis refroidie à une température voisine de 20°C et concentrée à sec sous pression réduite (2,7 kPa) à une température voisine de 40°C. Le résidu est additionné de 50 cm3 d'eau distillée et alcalinisé jusqu'à un pH voisin de 7 par addition d'une solution aqueuse 7,5N d'hydroxyde de sodium puis extrait par 3 fois 50 cm3 d'acétate d'éthyle. Les phases organiques sont réunies, séchées sur sulfate de magnésium, filtrées puis concentrées à sec sous pression réduite (2,7 kPa) à 40°C. On obtient ainsi 2,48 g d'hydroxy-2 [(phényl-1(S)] éthylamino-3 furyl-3 propionate-(2R,3S) de méthyle sous forme d'une huile jaune pâle.

L'hydroxy-3 (furyl-3)-4 [phényl-1-(S)] éthyl-1 azétidinone-2-(3R,4S) peut être préparé de la manière suivante :

A un mélange de 213 cm3 d'une solution aqueuse 1N d'hydroxyde de potassium et de 200 cm3 de tétrahydrofuranne on ajoute en 35 minutes, sous agitation et à une température voisine de 0°C, une solution de 9,24 g d'un mélange en proportion molaire 70/30 des deux diastéréoisomères d'acétoxy-3 (furyl-3)-4 [phényl-1-(S)] éthyl-1 azétidinone-2 forme A et forme B dans 200 cm3 de tétrahydrofuranne.

L'addition terminée le milieu réactionnel est agité à une température voisine de 0°C pendant 1 heure puis additionné de 200 cm3 d'une solution aqueuse saturée d'hydrogénocarbonate de sodium et de 200 cm3 d'eau distillée. La phase aqueuse est séparée par décantation et extraite par 3 fois 200 cm3 d'acétate d'éthyle. Les phases organiques sont réunies, séchées sur sulfate de magnésium, filtrées puis concentrées à sec sous pression réduite (2,7 kPa) à 40°C. On obtient ainsi 8,1 g d'une huile jaune que l'on cristallise dans 100 cm3 d'un mélange d'acétate d'éthyle et d'hexane (60-40 en volumes) pour donner 3,53 g d'hydroxy-3 (furyl-3)-4 [phényl-1-(S)] éthyl-1 azétidinone-2-(3R,4S) sous forme de cristaux blancs fondant à 100°C.

Le mélange en proportion molaire 70/30 des deux diastéréoisomères d'acétoxy-3 (furyl-3)-4 [phényl-1-(S)] éthyl-1 azétidinone-2 forme A et forme B peut être préparé de la manière suivante :

A une solution de 11,69 g de N-[(furyl-3) idène]-(phényl-1 éthylamine)-(S) dans 100 cm3 de toluène on ajoute, sous agitation et à une température voisine de -15°C, 15,5 cm3 de triéthylamine et additionne goutte à goutte, en 75 minutes et en se maintenant à cette température, 5,0 cm3 de chlorure d'acétoxy-2 acétyle. La solution obtenue est réchauffée jusqu'à une température voisine de 20°C et maintenue à cette température, sous agitation, pendant 16 heures puis additionnée de 300 cm3 d'une solution aqueuse 2,7N d'acide chlorhydrique. La phase organique est séparée par décantation, lavée par 2 fois 150 cm3 d'eau distillée puis par 150 cm3 d'une solution aqueuse saturée d'hydrogénocarbonate de sodium, séchée sur sulfate de magnésium, filtrée puis concentrée à sec sous pression réduite (2,7 kPa) à 40°C. On obtient ainsi 12,8 g d'une huile brune que l'on purifie par chromatographie sur 400 g de silice (0,04-0,063 mm) contenus dans une colonne de 5 cm de diamètre [éluant : cyclohexane-acétate d'éthyle (80-20 en volumes)]. Les fractions ne contenant que le produit cherché sont réunies et concentrées à sec sous pression réduite (0,27 kPa) à 40°C. On obtient ainsi 10,7 g d'un mélange en proportion molaire 70/30 des deux diastéréoisomères d'acétoxy-3 (furyl-3)-4 [phényl-1-(S)] éthyl-1 azétidinone-2 forme A et forme B sous forme d'une huile jaune.

La N-[(furyl-3) idène]-(phényl-1 éthylamine)-(S) peut être préparée de la manière suivante :

A une solution de 19,22 g de furannecarbaldéhyde-3 dans 165 cm3 de dichlorométhane on ajoute, sous agitation et à une température voisine de -15°C, 25,4 cm3 de phényl-1 éthylamine-(S) et 5 g de tamis moléculaire 4Å. Le mélange réactionnel est réchauffé jusqu'à une température voisine de 20°C et maintenu à cette température, sous agitation, pendant 24 heures puis filtré sur verre fritté garni de célite. Le verre fritté est lavé par 3 fois 30 cm3 de dichlorométhane et les filtrats sont réunis puis concentrés à sec sous pression réduite (2,7 kPa) à une température voisine de 40°C. On obtient ainsi 39,85 g de N-[(furyl-3) idène]-(phényl-1 éthylamine)-(S) sous forme d'une huile brune.

### EXEMPLE 3

Une solution de 0,60 g de tert-butoxycarbonylamino-3 hydroxy-2 (thiazolyl-4)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans un mélange de 8 cm3 de méthanol et de 8 cm3 d'acide acétique est chauffée sous agitation et sous atmosphère d'argon jusqu'à une température voisine de 60°C puis additionnée de 1,2 g de zinc en poudre. Le mélange réactionnel est ensuite agité pendant 15 minutes à 60°C puis refroidi à une température voisine de 20°C et filtré sur verre fritté garni de célite. Le verre fritté est lavé par 3 fois 10 cm3 de méthanol et les filtrats sont réunis puis concentrés à sec sous pression réduite (2,7 kPa) à une température voisine de 40°C.

Le résidu est additionné de 10 cm3 d'eau distillée et le solide cristallisé est séparé par filtration, lavé par 4 fois 5 cm3 d'eau distillée et séché sous pression réduite (0,27 kPa) à 20°C pendant 16 heures. On obtient 0,35 g d'une meringue blanche que l'on purifie par chromatographie sur gel de silice déposé sur plaque (gel de 0,25 mm d'épaisseur ; plaques de 20 x 20 cm) par fractions de 10 mg. Après localisation aux rayons U.V. de la zone correspondant au produit cherché adsorbé, cette zone est grattée et la silice recueillie est lavée sur verre fritté par 10 fois 10 cm3 de dichlorométhane et par 5 fois 5 cm3 de méthanol. Les filtrats sont réunis et concentrés à sec sous pression réduite (0,27 kPa) à 40°C pendant 16 heures. On obtient ainsi 0,135 g de tert-butoxycarbonylamino-3 (thiazolyl-4)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : []²⁰_{D} = -32 (c = 0,57 ; méthanol)
- spectre de R.M.N. : (400 MHz; CDCl₃ ; δ en ppm) 1,12 (s, 3H : -CH₃ 16 ou 17) ; 1,24 (s, 3H : -CH₃ 16 ou 17) ; 1,40 [s, 9H : -C(CH₃)₃] ; 1,70 (s, 1H : -OH 1) ; 1,78 (s, 3H : -CH₃ 19) ; 1,87 [mt, 1H : -(CH)-H 6] ; 1,90 (s, 3H : -CH₃ 18) ; 2,30 (ab dédoublé, J = 16 et 9 Hz, 2H : -CH₂- 14) ; 2,45 (s, 3H : -COCH₃) ; 2,60 [mt, 1H : -(CH)-H 6] ; 3,94 (d, J = 7 Hz, 1H : -H 3) ; 4,20 (d, J = 8 Hz, 1H : -(CH)-H 20) ; 4,20 (mf, 1H: -OH 10) ; 4,25 (dd large, J = 11,5 et 7,5 Hz, 1H : -H 7) ; 4,34 [d, J = 8 Hz, 1H : -(CH)-H 20] ; 4,88 (s large, 1H : -H 2') ; 4,97 (d large, J = 10 Hz, 1H : -H 5) ; 5,20 (s, 1H: -H 10) ; 5,47 (d large, J = 10 Hz, 1H: -H 3') ; 5,68 (d, J = 10 Hz, 1H: -CONH-) ; 5,70 (d, J = Hz, 1H: -H 2) ; 6,20 (t, J = 9 Hz, 1H : -H 13) ; 7,32 [s large, 1H : (-H 5)-thiazolyl-4] ; 7,50 [t, J = 7,5 Hz, 2H : -OCOC₆H₅(-H 3 et -H 5)] ; 7,60 [t, J = 7,5 Hz, 1H : -OCOC₆H₅(-H 4)]; 8,11 [d, J = 7,5 Hz, 2H : -OCOC₆H₅(-H 2 et -H 6)] ; 8,80 [d, J = 1,5 Hz, 1H : (-H2)-thiazolyl-4].

En opérant comme dans l'exemple 2, à partir de matières premières convenables, sont préparés les intermédiaires suivants :
- le tert-butoxycarbonylamino-3 hydroxy-2 (thiazolyl-4)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.
- l'amino-3 hydroxy-2 (thiazolyl-4)-3 propionate-(2R,3S) d'acétoxy-4 benzoyloxy-2a époxy-5β,20 hydroxy-1 oxo-9 bis-(trichloro-2,2,2 éthoxy)carbonyloxy-7β,10β taxène-11 yle-13α sous forme d'une meringue blanche.
- le tert-butoxycarbonyl-3 (méthoxy-4 phényl)-2 (thiazolyl-4)-4 oxazolidine-carboxylate-5-(2RS,4S,5R) d'acétoxy-4, benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.
- l'acide tert-butoxycarbonyl-3 (méthoxy-4 phényl)-2 (thiazolyl-4)-4 oxazolidine-carboxylique-5-(2RS,4S,5R) sous forme d'une meringue blanche.
- le tert-butoxycarbonyl-3 (méthoxy-4 phényl)-2 (thiazolyl-4)-4 oxazolidine-carboxylate-5-(2RS,4S,5R) de méthyle sous forme d'une huile jaune pâle.
- le tert-butoxycarbonylamino-3 hydroxy-2 (thiazolyl-4)-3 propionate-(2R,3S) de méthyle sous forme d'une meringue blanche.
- l'amino-3 hydroxy-2 (thiazolyl-4)-3 propionate-(2R,3S) de méthyle sous forme d'une huile jaune pâle.
- l'hydroxy-2 [(phényl-1(S)]éthylamino-3 thiazolyl-4 propionate-(2R,3S) de méthyle sous forme d'une huile jaune pâle.
- l'hydroxy-3 (thiazolyl-4)-4 [phényl-1-(S)]éthyl-1 azétidinone-2-(3R,4S) sous forme d'une meringue blanche.
- le mélange en proportion molaire: 70/30 des deux diastéréoisomères d'acétoxy-3 (thiazolyl-4)-4 [phényl-1-(S)]éthyl-1 azétidinone-2 forme A et forme B sous forme d'une huile jaune pâle.
- la N-[(thiazolyl4)idène]-(phényl-1 éthylamine)-(S) sous forme d'une huile jaune.
- le thiazolecarbaldéhyde-4 peut être préparé selon la méthode décrite par A. Dondoni et al., Synthesis., 1987, 998-1001.
- la (méthoxy-4 phényl)-1 éthylamine-(S) peut être préparée selon la méthode décrite par H.O. Bernhard et al., Helv. Chim. Acta., 1973, 56(4), 1266-1303.

Les nouveaux produits de formule générale (I) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérélisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons(α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomusine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Procédé de préparation de produits de formule générale : 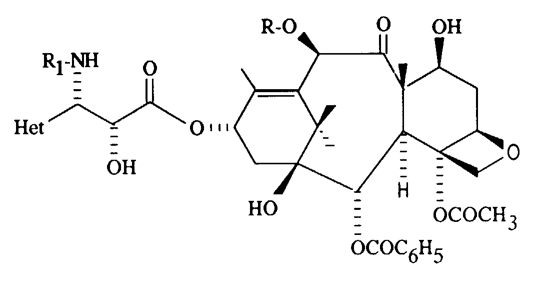 dans laquelle :
R représente un atome d'hydrogène ou un radical acétyle,
R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle, et
Het représente un radical hétérocyclyle aromatique ayant 5 chaînons et contenant un ou plusieurs hétéroatomes, identiques ou différents, choisis parmi l'azote, l'oxygène et le soufre, éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbo-nylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone, caractérisé en ce que l'on traite en milieu acide un produit de formule générale : 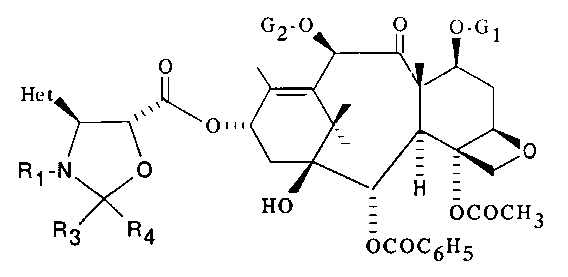 dans laquelle Het et R₁ sont définis comme précedemment, G₁ représente un groupement protecteur de la fonction hydroxy, G₂ représente un radical acétyle ou un groupement protecteur de la fonction hydroxy, R₃ représente un atome d'hydrogène ou un radical alcoxy ou un radical aryle éventuellement substitué et R₄ représente un atome d'hydrogène, pour obtenir un produit de formule générale : 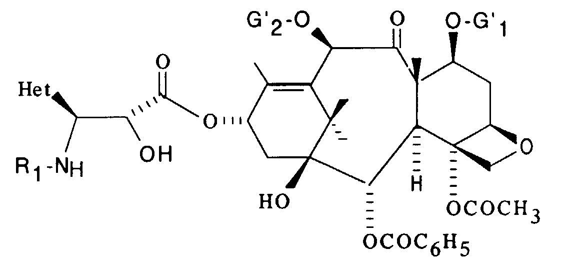 dans laquelle Het et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, puis remplace, si nécessaire, les groupements protecteurs G'₁ et G'₂ par des atomes d'hydrogène et isole le produit ainsi obtenu.

2. Procédé de préparation selon la revendication 1 de produits pour lesquels
R représente un atome d'hydrogène ou un radical acétyle,
R₁ représente un radical benzoyle ou un radical R₂-O-CO dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, nitro, carboxy ou alcoxycarbonyle dont la partie alcoyle contient 1 à 4 atomes de carbone,
- ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Het représente un radical hétérocyclique aromatique ayant 5 chaînons et contenant un ou plusieurs atomes, identiques ou différents, choisis parmi les atomes d'azote, d'oxygène ou de soufre, tel que le thiophène, le thiazole, le furanne, le pyrrole, l'imidazole, l'isoxazole ou le pyrazole, éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbo-nylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

3. Procédé de préparation selon la revendication 1 de produits pour lesquels R représente un atome d'hydrogène ou un radical acétyle, R₁ représente un radical benzoyle ou un radical R₂-O-CO- dans lequel R₂ représente un radical t.butyle et Het représente un radical thiényl-2 ou -3 ou furyl-2 ou -3.

4. Procédé selon les revendications 1, 2 ou 3 caractérisé en ce que le traitement acide est effectué au moyen d'un acide minéral ou organique dans un solvant organique à une température comprise entre -10 et 60°C.

5. Procédé selon la revendication 4 caractérisé en ce que l'acide est choisi parmi les acides chlorhydrique, sulfurique, acétique, méthanesulfonique, trifluorométhanesulfonique et p.toluènesulfonique utilisés seuls ou en mélange.

6. Procédé selon la revendication 4 caractérisé en ce que le solvant est choisi parmi les alcools, les éthers, les esters, les hydrocarbures aliphatiques halogénés, les hydrocarbures aromatiques et les nitriles.

7. Procédé selon les revendications 1, 2 ou 3 caractérisé en ce que les groupements protecteurs des fonctions hydroxy sont choisis parmi les radicaux trichloro-2,2,2 éthoxycarbonyle, (trichlorométhyl-2 propoxy)-2 carbonyle, trialkylsilyle, dialkylarylsilyle, alkyldiarylsilyle et triarylsilyle dans lesquels les radicaux alkyles contiennent 1 à 4 atomes de carbone et les radicaux aryles sont des radicaux phényles.

8. Procédé selon la revendication 7 caractérisé en ce que, lorsque G₁ et/ou G₂ représentent un radical silylé, leur remplacement par des atomes d'hydrogène s'effectue simultanément avec le remplacement du groupement protecteur de la chaîne latérale par traitement en milieu acide.

9. Procédé selon la revendication 7 caractérisé en ce que, lorsque G'₁ et/ou G'₂ représentent un radical trichloro-2,2,2 éthoxycarbonyle ou (trichlorométhyl-2 propoxy)-2 carbonyle, leur remplacement par un atome d'hydrogène est effectué par le zinc, éventuellement associé à du cuivre, en présence d'acide acétique à une température comprise entre 20 et 60°C ou bien au moyen d'un acide minéral ou organique en solution dans un alcool aliphatique ou dans un ester aliphatique en présence de zinc éventuellement associé à du cuivre.

10. Procédé selon l'une des revendications 1, 2 ou 3 caractérisé en ce que l'on traite en milieu acide un produit de formule générale : 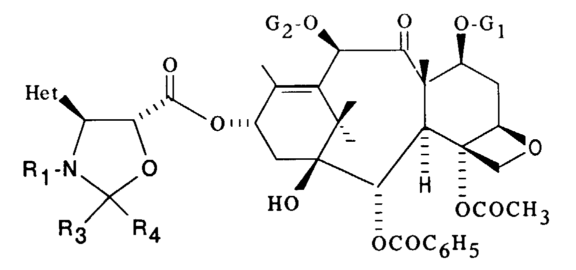 dans laquelle Het et R₁ sont définis comme dans l'une des revendications 1, 2 ou 3, G₁ représente un groupement protecteur de la fonction hydroxy, G₂ représente un radical acétyle ou un groupement protecteur de la fonction hydroxy, R₃ et R₄, identiques ou différents, représentent un radical alcoyle contenant 1 à 4 atomes de carbone ou un radical aralcoyle dont la partie alcoyle contient 1 à 4 atomes de carbone ou un radical aryle, ou bien R₃ représente un radical trihalométhyle ou un radical phényle substitué par un radical trihalométhyle et R₄ représente un atome d'hydrogène, ou bien R₃ et R₄ forment ensemble avec l'atome de carbone auquel ils sont liés un cycle ayant 4 à 7 chaînons, pour obtenir un produit de formule générale : 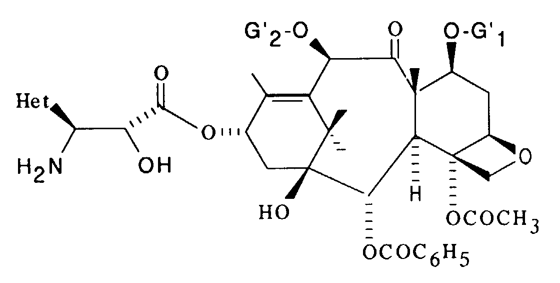 dans laquelle Het est défini comme dans l'une des revendications 1, 2 ou 3, G'₁ représente un atome d'hydrogène ou un groupement protecteur de la fonction hydroxy et G'₂ représente un radical acétyle ou un groupement protecteur de la fonction hydroxy, qui est acylé au moyen de chlorure de benzoyle ou d'un dérivé réactif de formule générale :
R₂-O-CO-X
dans laquelle R₂ est défini comme dans l'une des revendications 1, 2 ou 3 et X représente un atome d'halogène ou un reste-O-R₂ ou -O-CO-O-R₂, pour obtenir un produit de formule générale : 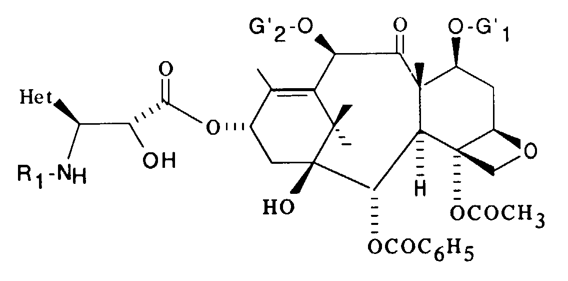 dans laquelle Het, R₁, G'₁ et G'₂ sont définis comme précédemment, puis remplace, si nécessaire, les groupements protecteurs G'₁ et G'₂ par des atomes d'hydrogène, et isole le produit obtenu.

11. Procédé selon la revendication 10 caractérisé en ce que le traitement acide est effectué au moyen d'un acide minéral ou organique dans un solvant organique à une température comprise entre -10 et 60°C.

12. Procédé selon la revendication 11 caractérisé en ce que l'acide est choisi parmi les acides chlorhydrique, sulfurique, acétique, méthanesulfonique, trifluorométhanesulfonique, p.toluènesulfonique et formique utilisés seuls ou en mélange.

13. Procédé selon la revendication 11 caractérisé en ce que le solvant est choisi parmi les alcools, les éthers, les esters, les hydrocarbures aliphatiques halogénés, les hydrocarbures aromatiques et les nitriles.

14. Procédé selon la revendication 10 caractérisé en ce que l'acylation est effectuée dans un solvant organique inerte en présence d'une base minérale ou organique.

15. Procédé selon la revendication 14 caractérisé en ce que le solvant organique inerte est choisi parmi les esters et les hydrocarbures aliphatiques halogénés.

16. Procédé selon l'une des revendications 13, 14 ou 15 caractérisé en ce que l'on opère à une température comprise entre 0 et 50°C.

17. Procédé selon la revendication 10 caractérisé en ce que le remplacement par des atomes d'hydrogène des groupements protecteurs G'₁ et éventuellement G'₂, lorsqu'ils représentent un radical trichloro-2,2,2 éthoxycarbonyle ou (trichlorométhyl-2 propoxy)-2 carbonyle, est effectué par le zinc, éventuellement associé à du cuivre, en présence d'acide acétique à une température comprise entre 30 et 60°C ou au moyen d'un acide minéral ou organique en solution dans un alcool aliphatique contenant 1 à 3 atomes de carbone ou dans un ester aliphatique en présence de zinc éventuellement associé à du cuivre.

18. Procédé de préparation d'un produit de formule générale : 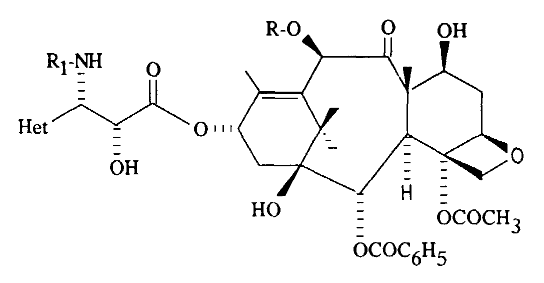 dans laquelle :
R représente un atome d'hydrogène ou un radical acétyle,
R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle, et
Het représente un radical hétérocyclyle aromatique éventuellement substitué ayant 5 chaînons et contenant un ou plusieurs hétéroatomes, identiques ou différents, choisis parmi l'azote, l'oxygène et le soufre,
caractérisé en ce que l'on estérifie la baccatine III ou la désacétyl-10 baccatine III protégée de formule générale : 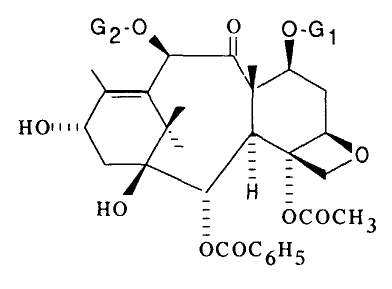 dans laquelle G₁ représente un groupement protecteur de la fonction hydroxy et G₂ représente un radical acétyle ou un groupement protecteur de la fonction hydroxy, au moyen d'un acide de formule générale : 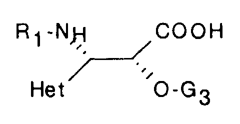 dans laquelle Het et R₁ sont définis comme dans l'une des revendications 1, 2 ou 3 et G₃ représente un groupement protecteur de la fonction hydroxy, ou d'un dérivé activé de cet acide, pour obtenir un produit de formule générale : 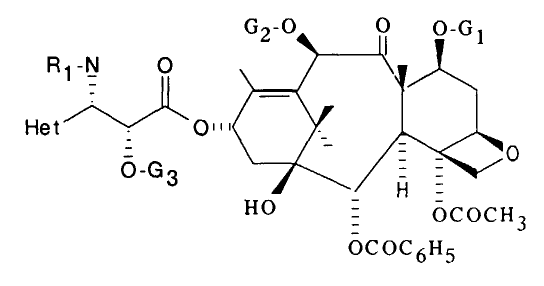 dans laquelle Het, R₁, G₁, G₂ et G₃ sont définis comme précédemment, dont on remplace les groupements protecteurs G₁, G₂ et G₃ par des atomes d'hydrogène, et isole le produit obtenu.

19. Procédé de préparation selon la revendication 18 de produits pour lesquels
R représente un atome d'hydrogène ou un radical acétyle,
R₁ représente un radical benzoyle ou un radical R₂-O-CO dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, nitro, carboxy ou alcoxycarbonyle dont la partie alcoyle contient 1 à 4 atomes de carbone,
- ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Het représente un radical hétérocyclique aromatique ayant 5 chaînons et contenant un ou plusieurs atomes, identiques ou différents, choisis parmi les atomes d'azote, d'oxygène ou de soufre, tel que le thiophène, le thiazole, le furanne, le pyrrole, l'imidazole, l'isoxazole ou le pyrazole, éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

20. Procédé de préparation selon la revendication 18 de produits pour lesquels R représente un atome d'hydrogène ou un radical acétyle, R₁ représente un radical benzoyle ou un radical R₂-O-CO- dans lequel R₂ représente un radical t.butyle et Het représente un radical thiényl-3 ou furyl-3.

21. Procédé selon les revendications 18, 19 ou 20 caractérisé en ce que l'estérification est effectuée au moyen de l'acide libre en opérant en présence d'un agent de condensation choisi parmi les carbodiimides et les carbonates réactifs et d'un agent d'activation choisi parmi les aminopyridines dans un solvant organique choisi parmi les éthers, les cétones, les esters, les nitriles, les hydrocarbures aliphatiques, les hydrocarbures aliphatiques halogénés et les hydrocarbures aromatiques à une température comprise entre -10 et 90°C.

22. Procédé selon les revendications 18, 19 ou 20 caractérisé en ce que l'estérification au moyen de l'anhydride est effectué en présence d'un agent d'activation choisi parmi les aminopyridines dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 90°C.

23. Procédé selon les revendications 18, 19 ou 20 caractérisé en ce que l'estérification est effectuée au moyen d'un halogénure ou d'un anhydride avec un acide aliphatique ou aromatique, éventuellement préparé in situ, en opérant en présence d'une base choisie parmi les amines aliphatiques tertiaires dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 80°C.

24. Procédé selon les revendications 18, 19 ou 20 caractérisé en ce que le remplacement des groupements protecteurs G₁, G₂ et G₃ par des atomes d'hydrogène est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre, lorsque G₁, G₂ et/ou G₃ représentent un radical trichloro-2,2,2 éthoxycarbonyle ou (trichlorométhyl-2 propoxy)-2 carbonyle, ou par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhydrique aqueux à une température comprise entre 0 et 40°C lorsque G₁, G₂ et/ou G₃ représentent un radical silylé.

25. Procédé selon les revendications 18, 19 ou 20 caractérisé en ce que, lorsque G₃ représente un radical -CH₂-Ph, on effectue d'abord le remplacement des groupements G₁ et G₂ par des atomes d'hydrogène dans les conditions de la revendication 24 avant de remplacer le groupement G₃ par hydrogénolyse.

26. Nouveaux dérivés du taxane de formule générale : 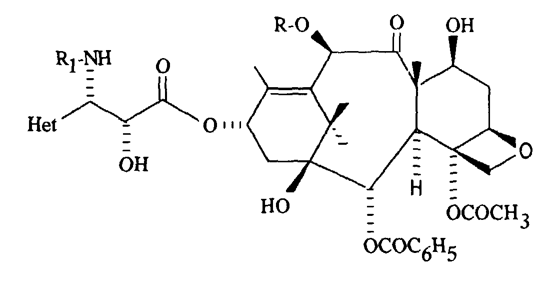 dans laquelle
R représente un atome d'hydrogène ou un radical acétyle,
R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle, et
Het représente un radical hétérocyclyle aromatique ayant 5 chaînons et contenant un ou plusieurs hétéroatomes, identiques ou différents, choisis parmi l'azote, l'oxygène et le soufre, éventuellement substitué par un ou plusieurs substituants, identiques ou différents, choisis parmi les atomes d'halogène et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbo-nylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone, à l'exception pour Het de représenter un radical furyl-2 ou thiényl-2 lorsque R₁ représente un radical benzoyle ou lorsque R₂ représente un alcoyle comportant de 1 à 6 atomes de carbone.

27. Nouveaux dérivés selon la revendication 26 pour lesquels
R représente un atome d'hydrogène ou un radical acétyle,
R₁ représente un radical benzoyle ou un radical R₂-O-CO dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, nitro, carboxy ou alcoxycarbonyle dont la partie alcoyle contient 1 à 4 atomes de carbone,
- ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Het représente un radical hétérocyclique aromatique ayant 5 chaînons et contenant un ou plusieurs atomes, identiques ou différents, choisis parmi les atomes d'azote, d'oxygène ou de soufre, tel que le thiophène, le thiazole, le furanne, le pyrrole, l'imidazole, l'isoxazole ou le pyrazole,éventuellement substitué par un ou plusieurs substituants, identiques ou différents, choisis parmi les atomes d'halogène (fluor, chlore) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbo-nylamino contenant 1 à 4 atomes de carbone, acyle contenant 1 à 4 atomes de carbone, arylcarbonyle dont la partie aryle contient 6 à 10 atomes de carbone, cyano, nitro, hydroxy, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone, à l'exception pour Het de représenter un radical thiényl-2 ou furyl-2 lorsque R₁ représente un radical benzoyle ou lorsque R₂ représente un radical alcoyle contenant 1 à 6 atomes de carbone.

28. Nouveaux dérivés selon la revendication 26 pour lesquels R représente un atome d'hydrogène ou un radical acétyle, R₁ représente un radical benzoyle ou un radical R₂-O-CO- dans lequel R₂ représente un radical t.butyle et Het représente un radical thiényl-3 ou furyl-3.

29. Composition pharmaceutique caractérisée en ce qu'elle contient au moins un produit selon les revendications 26, 27 ou 28 en association avec un ou plusieurs produits pharmaceutiquement acceptables qu'ils soient inertes ou physiologiquement actifs.

## Patentansprüche

1. Verfahren zur Herstellung von Produkten der allgemeinen Formel 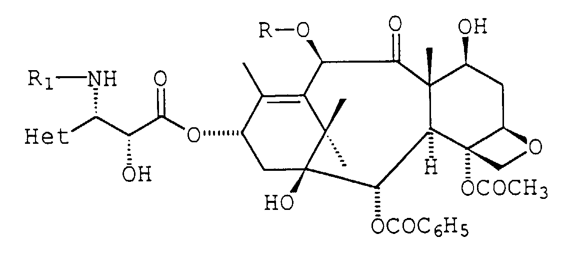 in der
R ein Wasserstoffatom oder einen Rest Acetyl darstellt,
R₁ einen Rest Benzoyl oder einen Rest R₂-O-CO- bedeutet, worin R₂ ein Rest Alkyl, Alkenyl, Alkinyl, Cycloalkyl, Cycloalkenyl, Bicycloalkyl, Phenyl oder Heterocyclyl ist, und
Het einen aromatischen Rest Heterocyclyl mit 5 Ringgliedern darstellt, der ein oder mehrere, gleiche oder verschiedene Heteroatome enthält, ausgewählt unter Stickstoff, Sauerstoff und Schwefel, gegebenenfalls substituiert durch einen oder mehrere, gleiche oder verschiedene Substituenten, ausgewählt unter den Halogenatomen (Fluor, Chlor) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Hydroxy, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält oder Alkoxycarbonyl, dessen Alkoxyteil 1 bis 4 Kohlenstoffatome enthält, dadurch gekennzeichnet, daß man ein Produkt der allgemeinen Formel 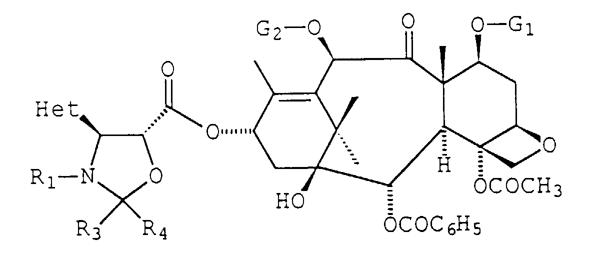 im sauren Medium behandelt, worin Het und R₁ wie oben definiert sind, G₁ eine Schutzgruppe für die Hydroxyfunktion darstellt und G₂ einen Rest Acetyl oder eine Schutzgruppe für die Hydroxyfunktion darstellt, R₃ ein Wasserstoffatom oder einen Rest Alkoxy oder einen gegebenenfalls substituierten Rest Aryl bedeutet und R₄ ein Wasserstoffatom ist, um ein Produkt der allgemeinen Formel 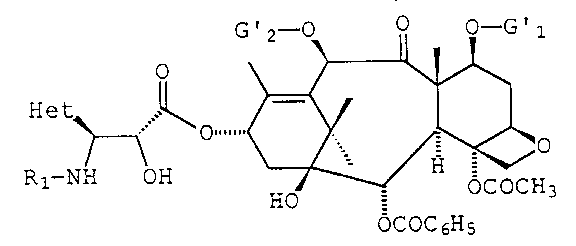 zu erhalten, in der Het und R₁ wie oben definiert sind, G'₁ ein Wasserstoffatom oder eine Schutzgruppe für die Hydroxyfunktion darstellt und G'₂ ein Wasserstoffatom oder einen Rest Acetyl oder eine Schutzgruppe für die Hydroxyfunktion darstellt, und man anschließend, wenn erforderlich, die Schutzgruppen G'₁ und G'₂ durch Wasserstoffatome ersetzt und das auf diese Weise erhaltene Produkt isoliert.

2. Verfahren zur Herstellung nach Anspruch 1 von Produkten, worin
R ein Wasserstoffatom oder einen Rest Acetyl darstellt,
R₁ einen Rest Benzoyl oder einen Rest R₂-O-CO- bedeutet, worin R₂ darstellt:
- einen geraden oder verzweigten Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere, gleiche oder verschiedene Substituenten, ausgewählt unter den Halogenatomen und den Resten Hydroxy, Alkoxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Nitro, Carboxy oder Alkoxycarbonyl, dessen Alkylteil 1 bis 4 Kohlenstoffatome enthält,
- oder einen Rest Phenyl, gegebenenfalls substituiert durch einen oder mehrere, gleiche oder verschiedene Reste, ausgewählt unter den Resten Alkyl mit 1 bis 4 Kohlenstoffatomen oder Alkoxy mit 1 bis 4 Kohlenstoffatomen,
- oder einen gesättigten oder nicht gesättigten Rest Stickstoff-Heterocyclyl mit 5 oder 6 Ringgliedern, gegebenenfalls substituiert durch einen oder mehrere Reste Alkyl mit 1 bis 4 Kohlenstoffatomen,
mit der Maßgabe, daß die Reste Cycloalkyl, Cycloalkenyl oder Bicycloalkyl gegebenenfalls durch einen oder mehrere Reste Alkyl mit 1 bis 4 Kohlenstoffatomen substituiert sein können, und
Het einen aromatischen Rest Heterocyclyl mit 5 Ringgliedern darstellt, der ein oder mehrere, gleiche oder verschiedene Atome enthält, ausgewählt unter den Atomen von Stickstoff, Sauerstoff oder Schwefel, wie Thiophen, Thiazol, Furan, Pyrrol, Imidazol, Isoxazol oder Pyrazol, gegebenenfalls substituiert durch einen oder mehrere, gleiche oder verschiedene Substituenten, ausgewählt unter den Halogenatomen (Fluor, Chlor) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Hydroxy, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält oder Alkoxycarbonyl, dessen Alkoxyteil 1 bis 4 Kohlenstoffatome enthält.

3. Verfahren zur Herstellung nach Anspruch 1 von Produkten, worin R ein Wasserstoffatom oder einen Rest Acetyl darstellt, R₁ einen Rest Benzoyl oder einen Rest R₂-O-CO- bedeutet, worin R₂ ein Rest tert.-Butyl ist und Het einen Rest 2-Thienyl oder 3-Thienyl oder 2-Furyl oder 3-Furyl darstellt.

4. Verfahren nach Anspruch 1, 2 oder 3, dadurch gekennzeichnet, daß die saure Behandlung mit Hilfe einer Mineralsäure oder organischen Säure in einem organischen Lösungsmittel bei einer Temperatur zwischen -10 °C und 60 °C durchgeführt wird.

5. Verfahren nach Anspruch 4, dadurch gekennzeichnet, daß die Säure unter Chlorwasserstoffsäure, Schwefelsäure, Essigsäure, Methansulfonsäure, Trifluormethan-sulfonsäure und para-Toluolsulfonsäure ausgewählt wird, allein verwendet oder in Mischung.

6. Verfahren nach Anspruch 4, dadurch gekennzeichnet, daß das Lösungsmittel unter den Alkoholen, den Ethern, den Estern, den halogenierten aliphatischen Kohlenwasserstoffen, den aromatischen Kohlenwasserstoffen und den Nitrilen ausgewählt wird.

7. Verfahren nach Anspruch 1, 2 oder 3, dadurch gekennzeichnet, daß die Schutzgruppen für die Hydroxyfunktionen unter den Resten 2,2,2-Trichlor-ethoxycarbonyl, 2-(2-Trichlormethyl-propoxy)-carbonyl, Trialkylsilyl, Dialkylarylsilyl, Alkyldiarylsilyl und Triarylsilyl ausgewählt werden, worin die Reste Alkyl 1 bis 4 Kohlenstoffatome enthalten und die Reste Aryl Phenylreste sind.

8. Verfahren nach Anspruch 7, dadurch gekennzeichnet, daß in dem Fall, wo G₁ und/oder G₂ einen Rest Silyl darstellen, ihr Austausch durch Wasserstoffatome gleichzeitig mit dem Austausch der Schutzgruppe für die Seitenkette mittels Behandlung im sauren Medium erfolgt.

9. Verfahren nach Anspruch 7, dadurch gekennzeichnet, daß in dem Fall, wo G'₁ und/oder G'₂ einen Rest 2,2,2-Trichlor-ethoxycarbonyl oder 2-(2-Trichlormethyl-propoxy)-carbonyl darstellen, ihr Austausch durch ein Wasserstoffatom mit Hilfe von Zink, gegebenenfalls assoziiert mit Kupfer, in Anwesenheit von Essigsäure bei einer Temperatur zwischen 20 °C und 60 °C durchgeführt wird, oder auch mit Hilfe einer Mineralsäure oder organischen Säure in Lösung eines aliphatischen Alkohols oder in einem aliphatischen Ester in Anwesenheit von Zink, gegebenenfalls assoziiert mit Kupfer.

10. Verfahren nach einem der Ansprüche 1, 2 oder 3, dadurch gekennzeichnet, daß man ein Produkt der allgemeinen Formel 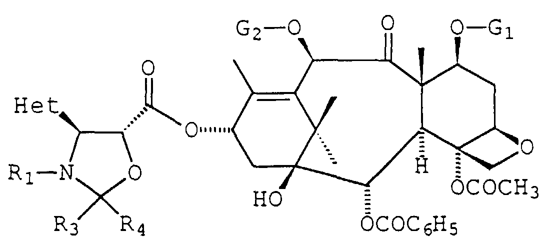 im sauren Medium behandelt, worin Het und R₁ wie in einem der Ansprüche 1, 2 oder 3 definiert sind, G₁ eine Schutzgruppe für die Hydroxyfunktion darstellt und G₂ einen Rest Acetyl oder eine Schutzgruppe für die Hydroxyfunktion darstellt, R₃ und R₄, gleich oder verschieden, einen Rest Alkyl mit 1 bis 4 Kohlenstoffatomen oder einen Rest Aralkyl, dessen Alkylteil 1 bis 4 Kohlenstoffatome enthält, oder einen Rest Aryl, bedeuten, oder auch R₃ einen Rest Trihalomethyl oder einen Rest Phenyl, substituiert durch einen Rest Trihalomethyl, darstellt und R₄ ein Wasserstoffatom ist, oder auch R₃ und R₄ zusammen mit dem Kohlenstoffatom, an das sie gebunden sind, einen Zyklus mit 4 bis 7 Ringgliedern bilden, um ein Produkt der allgemeinen Formel 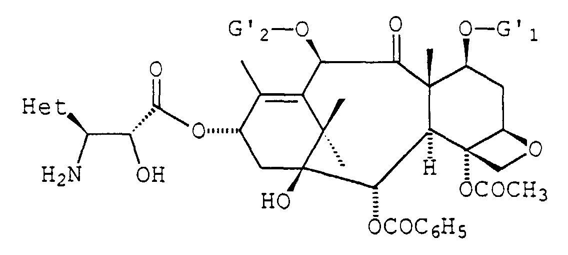 zu erhalten, in der Het wie in einem der Ansprüche 1, 2 oder 3 definiert ist, G'₁ ein Wasserstoffatom oder eine Schutzgruppe für die Hydroxyfunktion darstellt und G'₂ einen Rest Acetyl oder eine Schutzgruppe für die Hydroxyfunktion darstellt, das mit Hilfe von Benzoylchlorid oder einem reaktiven Derivat der allgemeinen Formel
R₂-O-CO-X
acyliert wird, worin R₂ wie in einem der Ansprüche 1, 2 oder 3 definiert ist und X ein Halogenatom oder einen Rest -O-R₂ oder -O-CO-O-R₂ darstellt, um ein Produkt der allgemeinen Formel 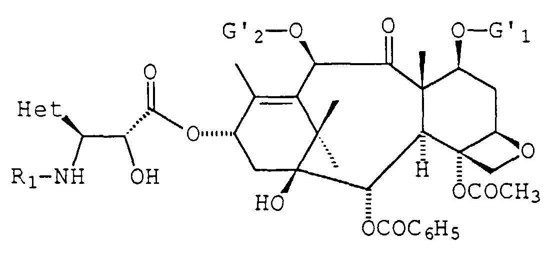 zu erhalten, in der Het, R₁, G'₁ und G'₂ wie oben definiert sind, und man anschließend, wenn erforderlich, die Schutzgruppen G'₁ und G'₂ durch Wasserstoffatome ersetzt und das auf diese Weise erhaltene Produkt isoliert.

11. Verfahren nach Anspruch 10, dadurch gekennzeichnet, daß die saure Behandlung mit Hilfe einer Mineralsäure oder organischen Säure in einem organischen Lösungsmittel bei einer Temperatur zwischen -10 °C und 60 °C durchgeführt wird.

12. Verfahren nach Anspruch 11, dadurch gekennzeichnet, daß die Säure unter Chlorwasserstoffsäure, Schwefelsäure, Essigsäure, Methansulfonsäure, Trifluormethan-sulfonsäure, para-Toluolsulfonsäure und Ameisensäure ausgewählt wird, allein verwendet oder in Mischung.

13. Verfahren nach Anspruch 11, dadurch gekennzeichnet, daß das Lösungsmittel unter den Alkoholen, den Ethern, den Estern, den halogenierten aliphatischen Kohlenwasserstoffen, den aromatischen Kohlenwasserstoffen und den Nitrilen ausgewählt wird.

14. Verfahren nach Anspruch 10, dadurch gekennzeichnet, daß die Acylierung in einem inerten organischen Lösungsmittel in Anwesenheit einer Mineralbase oder organischen Base durchgeführt wird.

15. Verfahren nach Anspruch 14, dadurch gekennzeichnet, daß das inerte organische Lösungsmittel unter den Estern und den halogenierten aliphatischen Kohlenwasserstoffen ausgewählt wird.

16. Verfahren nach einem der Ansprüche 13, 14 oder 15, dadurch gekennzeichnet, daß man bei einer Temperatur zwischen 0 °C und 50 °C arbeitet.

17. Verfahren nach Anspruch 10, dadurch gekennzeichnet, daß der Austausch der Schutzgruppen G'₁ und gegebenenfalls G'₂, wenn diese einen Rest 2,2,2-Trichlor-ethoxycarbonyl oder 2-(2-Trichlormethyl-propoxy)-carbonyl darstellen, durch Wasserstoffatome, mit Hilfe von Zink, gegebenenfalls assoziiert mit Kupfer, in Anwesenheit von Essigsäure bei einer Temperatur zwischen 30 °C und 60 °C durchgeführt wird, oder auch mit Hilfe einer Mineralsäure oder organischen Säure in Lösung eines aliphatischen Alkohols mit 1 bis 3 Kohlenstoffatomen oder in einem aliphatischen Ester in Anwesenheit von Zink, gegebenenfalls assoziiert mit Kupfer.

18. Verfahren zur Herstellung eines Produktes der allgemeinen Formel 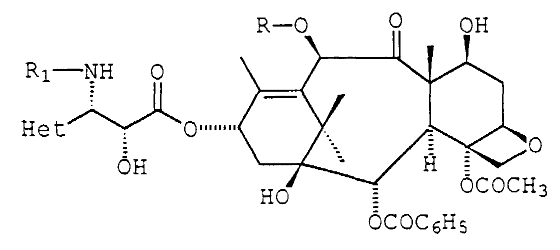 in der
R ein Wasserstoffatom oder einen Rest Acetyl darstellt,
R₁ einen Rest Benzoyl oder einen Rest R₂-O-CO- bedeutet, worin R₂ ein Rest Alkyl, Alkenyl, Alkinyl, Cycloalkyl, Cycloalkenyl, Bicycloalkyl, Phenyl oder Heterocyclyl ist, und
Het einen gegebenenfalls substituierten aromatischen Rest Heterocyclyl mit 5 Ringgliedern darstellt, der ein oder mehrere, gleiche oder verschiedene Heteroatome enthält, ausgewählt unter Stickstoff, Sauerstoff und Schwefel,
dadurch gekennzeichnet, daß man
geschütztes Baccatin III oder 10-Desacetyl-Baccatin III der allgemeinen Formel 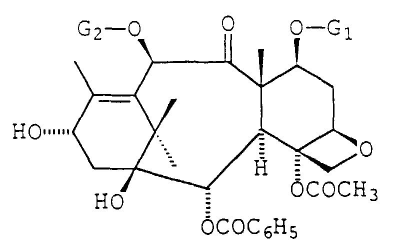 in der G₁ eine Schutzgruppe für die Hydroxyfunktion darstellt und G₂ einen Rest Acetyl oder eine Schutzgruppe für die Hydroxyfunktion darstellt, mit Hilfe einer Säure der allgemeinen Formel 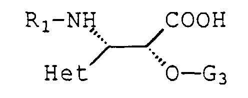 oder einem aktivierten Derivat dieser Säure verestert, worin Het und R₁ wie in einem der Ansprüche 1, 2 oder 3 definiert sind und G₃ eine Schutzgruppe für die Hydroxyfunktion darstellt, um ein Produkt der allgemeinen Formel 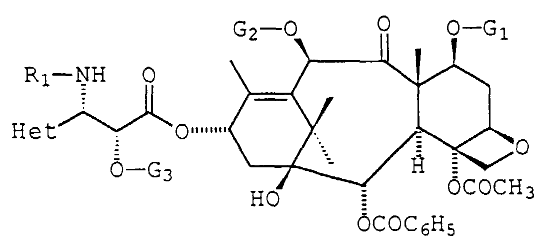 zu erhalten, worin Het, R₁, G₁, G₂ und G₃ wie oben definiert sind, bei dem man die Schutzgruppen G₁, G₂ und G₃ durch Wasserstoffatome ersetzt und das erhaltene Produkt isoliert.

19. Verfahren zur Herstellung nach Anspruch 18 von Produkten, worin
R ein Wasserstoffatom oder einen Rest Acetyl darstellt,
R₁ einen Rest Benzoyl oder einen Rest R₂-O-CO- bedeutet, worin R₂ darstellt :
- einen geraden oder verzweigten Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere, gleiche oder verschiedene Substituenten, ausgewählt unter den Halogenatomen und den Resten Hydroxy, Alkoxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Nitro, Carboxy oder Alkoxycarbonyl, dessen Alkylteil 1 bis 4 Kohlenstoffatome enthält,
- oder einen Rest Phenyl, gegebenenfalls substituiert durch einen oder mehrere, gleiche oder verschiedene Reste, ausgewählt unter den Resten Alkyl mit 1 bis 4 Kohlenstoffatomen oder Alkoxy mit 1 bis 4 Kohlenstoffatomen,
- oder einen gesättigten oder nicht gesättigten Rest Stickstoff-Heterocyclyl mit 5 oder 6 Ringgliedern, gegebenenfalls substituiert durch einen oder mehrere Reste Alkyl mit 1 bis 4 Kohlenstoffatomen,
mit der Maßgabe, daß die Reste Cycloalkyl, Cycloalkenyl oder Bicycloalkyl gegebenenfalls durch einen oder mehrere Reste Alkyl mit 1 bis 4 Kohlenstoffatomen substituiert sein können, und
Het einen aromatischen Rest Heterocyclyl mit 5 Ringgliedern darstellt, der ein oder mehrere, gleiche oder verschiedene Atome enthält, ausgewählt unter den Atomen von Stickstoff, Sauerstoff oder Schwefel, wie Thiophen, Thiazol, Furan, Pyrrol, Imidazol, Isoxazol oder Pyrazol, gegebenenfalls substituiert durch einen oder mehrere, gleiche oder verschiedene Substituenten, ausgewählt unter den Halogenatomen (Fluor, Chlor) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Hydroxy, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält oder Alkoxycarbonyl, dessen Alkoxyteil 1 bis 4 Kohlenstoffatome enthält.

20. Verfahren zur Herstellung nach Anspruch 18 von Produkten, worin R ein Wasserstoffatom oder einen Rest Acetyl darstellt, R₁ einen Rest Benzoyl oder einen Rest R₂-O-CO- bedeutet, worin R₂ ein Rest tert.-Butyl ist und Het einen Rest 3-Thienyl oder 3-Furyl darstellt.

21. Verfahren nach Anspruch 18, 19 oder 20, dadurch gekennzeichnet, daß die Veresterung mit Hilfe von freier Säure durchgeführt wird, wobei man in Anwesenheit eines Kondensationsmittels, ausgewählt unter den Carbodiimiden und den reaktiven Carbonaten, und eines Aktivierungsmittels, ausgewählt unter den Aminopyridinen, in einem organischen Lösungsmittel, ausgewählt unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen, bei einer Temperatur zwischen -10 °C und 90 °C arbeitet.

22. Verfahren nach Anspruch 18, 19 oder 20, dadurch gekennzeichnet, daß die Veresterung mit Hilfe von Anhydrid durchgeführt wird, wobei man in Anwesenheit eines Aktivierungsmittels, ausgewählt unter den Aminopyridinen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, bei einer Temperatur zwischen 0 °C und 90 °C arbeitet.

23. Verfahren nach Anspruch 18, 19 oder 20, dadurch gekennzeichnet, daß die Veresterung mit Hilfe eines Halogenides oder eines Anhydrides mit einer aliphatischen oder aromatischen Säure durchgeführt wird, gegebenenfalls in situ hergestellt, wobei man in Anwesenheit einer Base, ausgewählt unter den tertiären aliphatischen Aminen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, bei einer Temperatur zwischen 0 °C und 80 °C arbeitet.

24. Verfahren nach Anspruch 18, 19 oder 20, dadurch gekennzeichnet, daß der Austausch der Schutzgruppen G₁, G₂ und G₃ durch Wasserstoffatome mit Hilfe einer Behandlung mit Zink, gegebenenfalls assoziiert mit Kupfer, in Anwesenheit von Essigsäure bei einer Temperatur zwischen 30 °C und 60 °C durchgeführt wird, oder auch mit Hilfe einer Mineralsäure oder organischen Säure wie Chlorwasserstoffsäure oder Essigsäure in Lösung eines aliphatischen Alkohols mit 1 bis 3 Kohlenstoffatomen oder in einem aliphatischen Ester wie Ethylacetat, Isopropylacetat oder n-Butylacetat in Anwesenheit von Zink, gegebenenfalls assoziiert mit Kupfer, wenn G₁, G₂ und/oder G₃ einen Rest 2,2,2-Trichlor-ethoxycarbonyl oder 2-(2-Trichlormethyl-propoxy)-carbonyl darstellen, oder durch Behandlung im sauren Medium wie beispielsweise Chlorwasserstoffsäure in Lösung eines aliphatischen Alkohols mit 1 bis 3 Kohlenstoffatomen (Methanol, Ethanol, Propanol, Isopropanol) oder wäßriger Fluorwasserstoffsäure bei einer Temperatur zwischen 0 °C und 40 °C, wenn G₁, G₂ und/oder G₃ einen Rest Silyl darstellen.

25. Verfahren nach Anspruch 18, 19 oder 20, dadurch gekennzeichnet, daß man in dem Fall, wo G₃ einen Rest -CH₂-Ph darstellt, zuerst den Austausch der Gruppen G₁ und G₂ durch Wasserstoffatome unter den Bedingungen von Anspruch 24 durchführt, bevor man die Gruppe G₃ mittels Hydrogenolyse austauscht.

26. Neue Taxan-Derivate der allgemeinen Formel 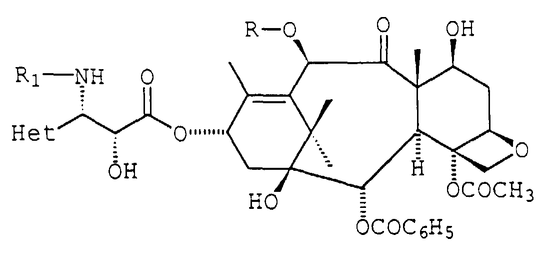 in der
R ein Wasserstoffatom oder einen Rest Acetyl darstellt,
R₁ einen Rest Benzoyl oder einen Rest R₂-O-CO- bedeutet, worin R₂ ein Rest Alkyl, Alkenyl, Alkinyl, Cycloalkyl, Cycloalkenyl, Bicycloalkyl, Phenyl oder Heterocyclyl ist, und
Het einen aromatischen Rest Heterocyclyl mit 5 Ringgliedern darstellt, der ein oder mehrere, gleiche oder verschiedene Heteroatome enthält, ausgewählt unter Stickstoff, Sauerstoff und Schwefel, gegebenenfalls substituiert durch einen oder mehrere, gleiche oder verschiedene Substituenten, ausgewählt unter den Halogenatomen und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Hydroxy, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält oder Alkoxycarbonyl, dessen Alkoxyteil 1 bis 4 Kohlenstoffatome enthält, mit der Ausnahme für Het, einen Rest 2-Furyl oder 2-Thienyl darzustellen, wenn R₁ einen Rest Benzoyl bedeutet oder wenn R₂ ein Rest Alkyl mit 1 bis 6 Kohlenstoffatomen ist.

27. Neue Derivate nach Anspruch 26, worin
R ein Wasserstoffatom oder einen Rest Acetyl darstellt,
R₁ einen Rest Benzoyl oder einen Rest R₂-O-CO- bedeutet, worin R₂ darstellt:
- einen geraden oder verzweigten Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere, gleiche oder verschiedene Substituenten, ausgewählt unter den Halogenatomen und den Resten Hydroxy, Alkoxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Nitro, Carboxy oder Alkoxycarbonyl, dessen Alkylteil 1 bis 4 Kohlenstoffatome enthält,
- oder einen Rest Phenyl, gegebenenfalls substituiert durch einen oder mehrere, gleiche oder verschiedene Reste, ausgewählt unter den Resten Alkyl mit 1 bis 4 Kohlenstoffatomen oder Alkoxy mit 1 bis 4 Kohlenstoffatomen,
- oder einen gesättigten oder nicht gesättigten Rest Stickstoff-Heterocyclyl mit 5 oder 6 Ringgliedern, gegebenenfalls substituiert durch einen oder mehrere Reste Alkyl mit 1 bis 4 Kohlenstoffatomen,
mit der Maßgabe, daß die Reste Cycloalkyl, Cycloalkenyl oder Bicycloalkyl gegebenenfalls durch einen oder mehrere Reste Alkyl mit 1 bis 4 Kohlenstoffatomen substituiert sein können, und
Het einen aromatischen Rest Heterocyclyl mit 5 Ringgliedern darstellt, der ein oder mehrere, gleiche oder verschiedene Atome enthält, ausgewählt unter den Atomen von Stickstoff, Sauerstoff oder Schwefel, wie Thiophen, Thiazol, Furan, Pyrrol, Imidazol, Isoxazol oder Pyrazol, gegebenenfalls substituiert durch einen oder mehrere, gleiche oder verschiedene Substituenten, ausgewählt unter den Halogenatomen (Fluor, Chlor) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Hydroxy, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält oder Alkoxycarbonyl, dessen Alkoxyteil 1 bis 4 Kohlenstoffatome enthält, mit der Ausnahme für Het, einen Rest 2-Furyl oder 2-Thienyl darzustellen, wenn R₁ einen Rest Benzoyl bedeutet oder wenn R₂ ein Rest Alkyl mit 1 bis 6 Kohlenstoffatomen ist.

28. Neue Derivate nach Anspruch 26, worin R ein Wasserstoffatom oder einen Rest Acetyl darstellt, R₁ einen Rest Benzoyl oder einen Rest R₂-O-CO- bedeutet, worin R₂ ein Rest tert.-Butyl ist und Het einen Rest 3-Thienyl oder 3-Furyl darstellt.

29. Pharmazeutische Zusammensetzungen, dadurch gekennzeichnet, daß sie mindestens ein Produkt nach den Ansprüchen 26, 27 oder 28 in Assoziation mit einem oder mehreren pharmazeutisch akzeptablen Produkten enthalten, die inert oder physiologisch aktiv sind.

## Claims

1. Process for the preparation of products of general formula: 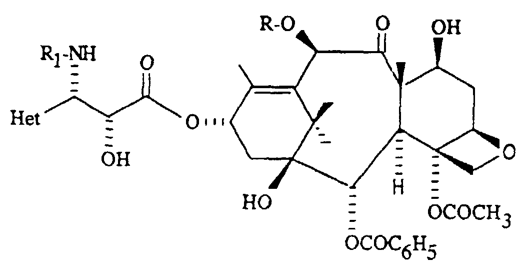 in which:
R represents a hydrogen atom or an acetyl radical,
R₁ represents a benzoyl radical or a radical R₂-O-CO- in which R₂ represents an alkyl, alkenyl, alkynyl, cycloalkyl, cycloalkenyl, bicycloalkyl, phenyl or heterocyclyl radical, and
Het represents an aromatic heterocyclyl radical having 5 members and containing one or a number of heteroatoms, which are identical or different, chosen from nitrogen, oxygen and sulphur, optionally substituted by one or a number of substituents, which are identical or different, chosen from the halogen atoms (fluorine, chlorine) and the alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, each alkyl part of which contains 1 to 4 carbon atoms, acylamino, the acyl part of which contains 1 to 4 carbon atoms, alkoxycarbonylamino, containing 1 to 4 carbon atoms, acyl, containing 1 to 4 carbon atoms, arylcarbonyl, the aryl part of which contains 6 to 10 carbon atoms, cyano, nitro, hydroxyl, carboxyl, carbamoyl, alkylcarbamoyl, the alkyl part of which contains 1 to 4 carbon atoms, dialkylcarbamoyl, each alkyl part of which contains 1 to 4 carbon atoms, or alkoxycarbonyl, the alkoxy part of which contains 1 to 4 carbon atoms, radicals, characterized in that a product of general formula: 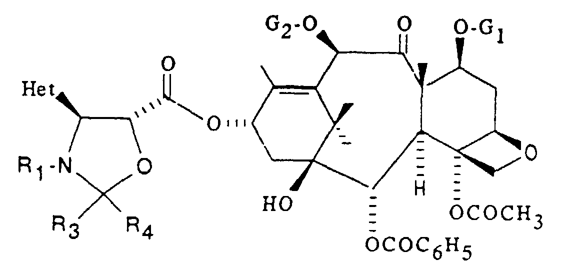 in which Het and R₁ are defined as above, G₁ represents a protective group of the hydroxyl functional group, G₂ represents an acetyl radical or a protective group of the hydroxyl functional group, R₃ represents a hydrogen atom or an alkoxy radical or an optionally substituted aryl radical and R₄ represents a hydrogen atom, is treated in acid medium to produce a product of general formula: 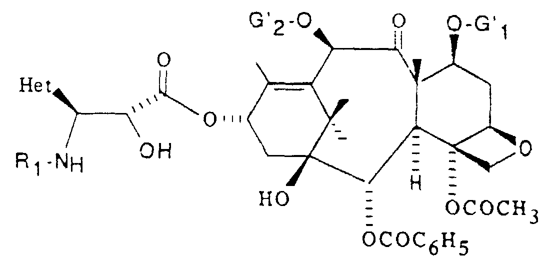 in which Het and R₁ are defined as above, G'₁ represents a hydrogen atom or a protective group of the hydroxyl functional group and G'₂ represents a hydrogen atom or an acetyl radical or a protective group of the hydroxyl functional group, and then, if necessary, the G'₁ and G'₂ protective groups are replaced by hydrogen atoms and the product thus obtained is isolated.

2. Process for the preparation according to claim 1 of products for which
R represents a hydrogen atom or an acetyl radical,
R₁ represents a benzoyl radical or a radical R₂-O-CO in which R₂ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted by one or a number of substituents, which are identical or different, chosen from the halogen atoms and the hydroxyl radical, alkoxy radical containing 1 to 4 carbon atoms, dialkylamino radical, each alkyl part of which contains 1 to 4 carbon atoms, piperidino radical, morpholino radical, 1-piperazinyl radical (optionally substituted in the 4-position by an alkyl radical containing 1 to 4 carbon atoms or by a phenylalkyl radical, the alkyl part of which contains 1 to 4 carbon atoms), cycloalkyl radical containing 3 to 6 carbon atoms, cycloalkenyl radical containing 4 to 6 carbon atoms, phenyl radical, cyano radical, nitro radical, carboxyl radical or alkoxycarbonyl radical, the alkyl part of which contains 1 to 4 carbon atoms,
- or a phenyl radical, optionally substituted by one or a number of radicals, which are identical or different, chosen from the alkyl radicals containing 1 to 4 carbon atoms or the alkoxy radicals containing 1 to 4 carbon atoms,
- or a saturated or unsaturated nitrogen-containing heterocyclyl radical containing 5 or 6 members, optionally substituted by one or a number of alkyl radicals containing 1 to 4 carbon atoms,
it being understood that the cycloalkyl, cycloalkenyl or bicycloalkyl radicals may optionally be substituted by one or a number of alkyl radicals containing 1 to 4 carbon atoms, and
Het represents an aromatic heterocyclic radical having 5 members and containing one or a number of atoms, which are identical or different, chosen from the nitrogen, oxygen or sulphur atoms, such as thiophene, thiazole, furan, pyrrole, imidazole, isoxazole or pyrazole, optionally substituted by one or a number of substituents, which are identical or different, chosen from the halogen atoms (fluorine, chlorine) and the alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, each alkyl part of which contains 1 to 4 carbon atoms, acylamino, the acyl part of which contains 1 to 4 carbon atoms, alkoxycarbonylamino, containing 1 to 4 carbon atoms, acyl, containing 1 to 4 carbon atoms, arylcarbonyl, the aryl part of which contains 6 to 10 carbon atoms, cyano, nitro, hydroxyl, carboxyl, carbamoyl, alkylcarbamoyl, the alkyl part of which contains 1 to 4 carbon atoms, dialkylcarbamoyl, each alkyl part of which contains 1 to 4 carbon atoms, or alkoxycarbonyl, the alkoxy part of which contains 1 to 4 carbon atoms, radicals.

3. Process for the preparation according to claim 1 of products for which R represents a hydrogen atom or an acetyl radical, R₁ represents a benzoyl radical or a radical R₂-O-CO- in which R₂ represents a t-butyl radical and Het represents a 2- or 3-thienyl or 2- or 3-furyl radical.

4. Process according to claim 1, 2 or 3, characterized in that the acid treatment is carried out by means of an inorganic or organic acid in an organic solvent at a temperature between -10 and 60°C.

5. Process according to claim 4, characterized in that the acid is chosen from hydrochloric, sulphuric, acetic, methanesulphonic, trifluoromethanesulphonic and p-toluenesulphonic acids, used alone or as a mixture.

6. Process according to claim 4, characterized in that the solvent is chosen from alcohols, ethers, esters, halogenated aliphatic hydrocarbons, aromatic hydrocarbons and nitriles.

7. Process according to claims 1, 2 or 3, characterized in that the protective groups of the hydroxyl functional groups are chosen from the 2,2,2-trichloroethoxycarbonyl, 2-(2-(trichloromethyl)propoxy) carbonyl, trialkylsilyl, dialkylarylsilyl, alkyldiarylsilyl and triarylsilyl radicals in which the alkyl radicals contain 1 to 4 carbon atoms and the aryl radicals are phenyl radicals.

8. Process according to claim 7, characterized in that, when G₁ and/or G₂ represent a silylated radical, their replacement by hydrogen atoms is carried out simultaneously with the replacement of the protective group of the side chain by treatment in acid medium.

9. Process according to claim 7, characterized in that, when G'₁ and/or G'₂ represent a 2,2,2-trichloroethoxycarbonyl or 2-(2-(trichloromethyl)propoxy)carbonyl radical, their replacement by a hydrogen atom is carried out with zinc, optionally in combination with copper, in the presence of acetic acid at a temperature between 20 and 60°C or else by means of an inorganic or organic acid in solution in an aliphatic alcohol or in an aliphatic ester in the presence of zinc, optionally in combination with copper.

10. Process according to one of claims 1, 2 or 3, characterized in that a product of general formula: 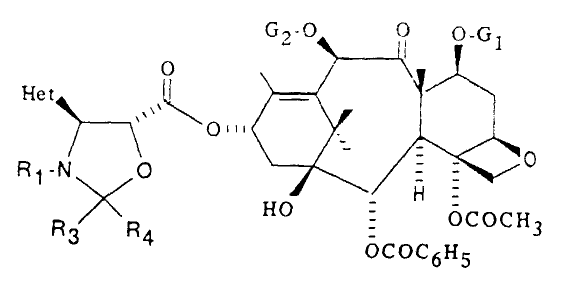 in which Het and R₁ are defined as in one of claims 1, 2 or 3, G₁ represents a protective group of the hydroxyl functional group, G₂ represents an acetyl radical or a protective group of the hydroxyl functional group, R₃ and R₄, which are identical or different, represent an alkyl radical containing 1 to 4 carbon atoms or an aralkyl radical, the alkyl part of which contains 1 to 4 carbon atoms, or an aryl radical, or else R₃ represents a trihalomethyl radical or a phenyl radical substituted by a trihalomethyl radical and R₄ represents a hydrogen atom, or else R₃ and R₄ form, together with the carbon atom to which they are bonded, a ring having 4 to 7 members, is treated in acid medium to produce a product of general formula: 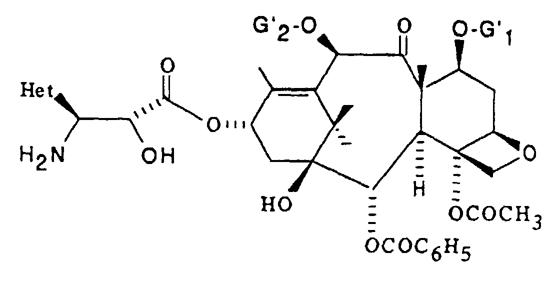 in which Het is defined as in one of claims 1, 2 or 3, G'₁ represents a hydrogen atom or a protective group of the hydroxyl functional group and G'₂ represents an acetyl radical or a protective group of the hydroxyl functional group, which is acylated by means of benzoyl chloride or of a reactive derivative of general formula:
R₂-O-CO-X
in which R₂ is defined as in one of claims 1, 2 or 3 and X represents a halogen atom or an -O-R₂ or -O-CO-O-R₂ residue, to produce a product of general formula: 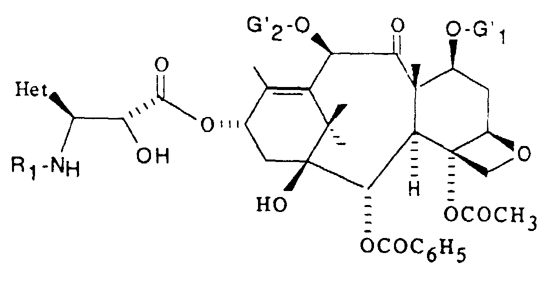 in which Het, R₁, G'₁ and G'₂ are defined as above, the G'₁ and G'₂ protective groups are then replaced, if necessary, by hydrogen atoms, and the product obtained is isolated.

11. Process according to claim 10, characterized in that the acid treatment is carried out by means of an inorganic or organic acid in an organic solvent at a temperature between -10 and 60°C.

12. Process according to claim 11, characterized in that the acid is chosen from hydrochloric, sulphuric, acetic, methanesulphonic, trifluoromethanesulphonic, p-toluenesulphonic and formic acids, used alone or as a mixture.

13. Process according to claim 11, characterized in that the solvent is chosen from alcohols, ethers, esters, halogenated aliphatic hydrocarbons, aromatic hydrocarbons and nitriles.

14. Process according to claim 10, characterized in that the acylation is carried out in an inert organic solvent in the presence of an inorganic or organic base.

15. Process according to claim 14, characterized in that the inert organic solvent is chosen from esters and halogenated aliphatic hydrocarbons.

16. Process according to claims 13, 14 or 15, characterized in that the reaction is carried out at a temperature between 0 and 50°C.

17. Process according to claim 10, characterized in that the replacement by hydrogen atoms of the G'₁ and optionally G'₂ protective groups, when they represent a 2,2,2-trichloroethoxycarbonyl or 2-(2-(trichloromethyl)propoxy)carbonyl radical, is carried out with zinc, optionally in combination with copper, in the presence of acetic acid at a temperature between 30 and 60°C or by means of an inorganic or organic acid in solution in an aliphatic alcohol containing 1 to 3 carbon atoms or in an aliphatic ester in the presence of zinc, optionally in combination with copper.

18. Process for the preparation of a product of general formula: 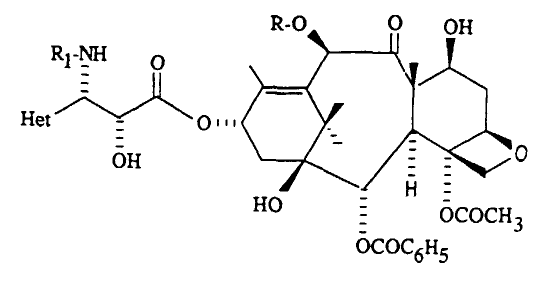 in which:
R represents a hydrogen atom or an acetyl radical,
R₁ represents a benzoyl radical or a radical R₂-O-CO- in which R₂ represents an alkyl, alkenyl, alkynyl, cycloalkyl, cycloalkenyl, bicycloalkyl, phenyl or heterocyclyl radical, and
Het represents an optionally substituted aromatic heterocyclyl radical having 5 members and containing one or a number of identical or different heteroatoms chosen from nitrogen, oxygen and sulphur,
characterized in that protected 10-deacetylbaccatin III or baccatin III of general formula : 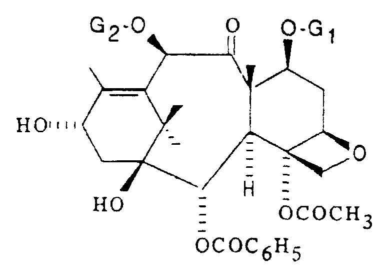 in which G₁ represents a protective group of the hydroxyl functional group and G₂ represents an acetyl radical or a protective group of the hydroxyl functional group, is esterified by means of an acid of general formula : 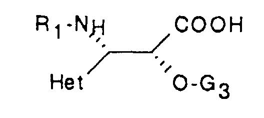 in which Het and R₁ are defined as in one of claims 1, 2 or 3 and G₃ represents a protective group of the hydroxyl functional group, or of an activated derivative of this acid, to produce a product of general formula: 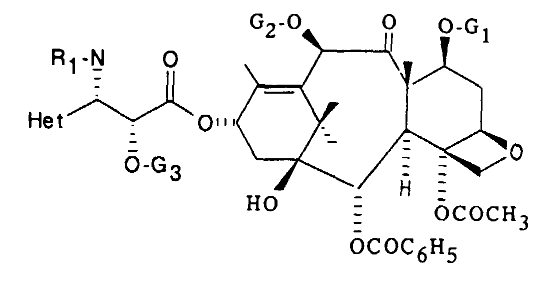 in which Het, R₁, G₁, G₂ and G₃ are defined as above, the G₁, G₂ and G₃ protective groups of which are replaced by hydrogen atoms, and the product obtained is isolated.

19. Process for the preparation according to claim 18 of products for which
R represents a hydrogen atom or an acetyl radical,
R₁ represents a benzoyl radical or a radical R₂-O-CO in which R₂ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted by one or a number of substituents, which are identical or different, chosen from the halogen atoms and the hydroxyl radical, alkoxy radical containing 1 to 4 carbon atoms, dialkylamino radical, each alkyl part of which contains 1 to 4 carbon atoms, piperidino radical, morpholino radical, 1-piperazinyl radical (optionally substituted in the 4-position by an alkyl radical containing 1 to 4 carbon atoms or by a phenylalkyl radical, the alkyl part of which contains 1 to 4 carbon atoms), cycloalkyl radical containing 3 to 6 carbon atoms, cycloalkenyl radical containing 4 to 6 carbon atoms, phenyl radical, cyano radical, nitro radical, carboxyl radical or alkoxycarbonyl radical, the alkyl part of which contains 1 to 4 carbon atoms,
- or a phenyl radical, optionally substituted by one or a number of radicals, which are identical or different, chosen from the alkyl radicals containing 1 to 4 carbon atoms or the alkoxy radicals containing 1 to 4 carbon atoms,
- or a saturated or unsaturated nitrogen-containing heterocyclyl radical containing 5 or 6 members, optionally substituted by one or a number of alkyl radicals containing 1 to 4 carbon atoms,
it being understood that the cycloalkyl, cycloalkenyl or bicycloalkyl radicals may optionally be substituted by one or a number of alkyl radicals containing 1 to 4 carbon atoms, and
Het represents an aromatic heterocyclic radical having 5 members and containing one or a number of atoms, which are identical or different, chosen from the nitrogen, oxygen or sulphur atoms, such as thiophene, thiazole, furan, pyrrole, imidazole, isoxazole or pyrazole, optionally substituted by one or a number of substituents, which are identical or different, chosen from the halogen atoms (fluorine, chlorine) and the alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, each alkyl part of which contains 1 to 4 carbon atoms, acylamino, the acyl part of which contains 1 to 4 carbon atoms, alkoxycarbonylamino, containing 1 to 4 carbon atoms, acyl, containing 1 to 4 carbon atoms, arylcarbonyl, the aryl part of which contains 6 to 10 carbon atoms, cyano, nitro, hydroxyl, carboxyl, carbamoyl, alkylcarbamoyl, the alkyl part of which contains 1 to 4 carbon atoms, dialkylcarbamoyl, each alkyl part of which contains 1 to 4 carbon atoms, or alkoxycarbonyl, the alkoxy part of which contains 1 to 4 carbon atoms, radicals.

20. Process for the preparation according to claim 18 of products for which R represents a hydrogen atom or an acetyl radical, R₁ represents a benzoyl radical or a radical R₂-O-CO- in which R₂ represents a t-butyl radical and Het represents a 3-thienyl or 3-furyl radical.

21. Process according to claims 18, 19 or 20, characterized in that the esterification is carried out by means of free acid, the reaction being carried out in the presence of a condensation agent chosen from carbodiimides and reactive carbonates and of an activating agent chosen from aminopyridines in an organic solvent chosen from ethers, ketones, esters, nitriles, aliphatic hydrocarbons, halogenated aliphatic hydrocarbons and aromatic hydrocarbons at a temperature between -10 and 90°C.

22. Process according to claims 18, 19 or 20, characterized in that the esterification by means of the anhydride is carried out in the presence of an activating agent chosen from aminopyridines in an organic solvent chosen from ethers, esters, ketones, nitriles, aliphatic hydrocarbons, halogenated aliphatic hydrocarbons and aromatic hydrocarbons at a temperature between 0 and 90°C

23. Process according to claims 18, 19 or 20, characterized in that the esterification is carried out by means of a halide or of an anhydride with an aliphatic or aromatic acid, optionally prepared in situ, the reaction being carried out in the presence of a base chosen from tertiary aliphatic amines in an organic solvent chosen from ethers, esters, ketones, nitriles, aliphatic hydrocarbons, halogenated aliphatic hydrocarbons and aromatic hydrocarbons at a temperature between 0 and 80°C.

24. Process according to claims 18, 19 or 20, characterized in that the replacement of the G₁, G₂ and G₃ protective groups by hydrogen atoms is carried out by treatment with zinc, optionally in combination with copper, in the presence of acetic acid at a temperature between 30 and 60°C or by means of an inorganic or organic acid such as hydrochloric acid or acetic acid in solution in an aliphatic alcohol containing 1 to 3 carbon atoms or an aliphatic ester such as ethyl acetate, isopropyl acetate or n-butyl acetate in the presence of zinc, optionally in combination with some copper, when G₁, G₂ and/or G₃ represent a 2,2,2-trichloroethoxycarbonyl or 2-(2-(trichloromethyl)propoxy)carbonyl radical, or by treatment in acid medium such as for example hydrochloric acid in solution in an aliphatic alcohol containing 1 to 3 carbon atoms (methanol, ethanol, propanol, isopropanol) or aqueous hydrofluoric acid at a temperature between 0 and 40°C when G₁, G₂ and/or G₃ represent a silylated radical.

25. Process according to claims 18, 19 or 20, characterized in that, when G₃ represents a -CH₂-Ph radical, the replacement is first carried out of the G₁ and G₂ groups by hydrogen atoms under the conditions of claim 24 before replacing the G₃ group by hydrogenolysis.

26. New taxane derivatives of general formula: 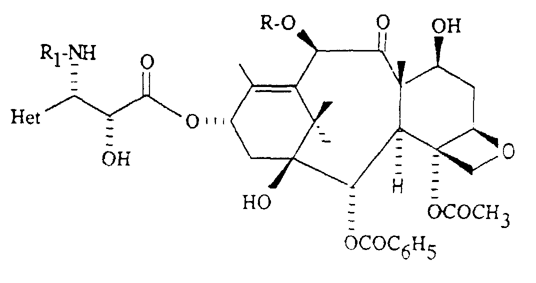 in which
R represents a hydrogen atom or an acetyl radical,
R₁ represents a benzoyl radical or a radical R₂-O-CO- in which R₂ represents an alkyl, alkenyl, alkynyl, cycloalkyl, cycloalkenyl, bicycloalkyl, phenyl or heterocyclyl radical, and
Het represents an aromatic heterocyclyl radical having 5 members and containing one or more identical or different heteroatoms chosen from nitrogen, oxygen and sulphur, optionally substituted by one or a number of substituents, which are identical or different, chosen from the halogen atoms and the alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, each alkyl part of which contains 1 to 4 carbon atoms, acylamino, the acyl part of which contains 1 to 4 carbon atoms, alkoxycarbonylamino, containing 1 to 4 carbon atoms, acyl, containing 1 to 4 carbon atoms, arylcarbonyl, the aryl part of which contains 6 to 10 carbon atoms, cyano, nitro, hydroxyl, carboxyl, carbamoyl, alkylcarbamoyl, the alkyl part of which contains 1 to 4 carbon atoms, dialkylcarbamoyl, each alkyl part of which contains 1 to 4 carbon atoms, or alkoxycarbonyl, the alkoxy part of which contains 1 to 4 carbon atoms, radicals, with the exception of Het representing a 2-furyl or 2-thienyl radical when R₁ represents a benzoyl radical or when R₂ represents an alkyl radical containing from 1 to 6 carbon atoms.

27. New derivatives according to claim 26 for which
R represents a hydrogen atom or an acetyl radical,
R₁ represents a benzoyl radical or a radical R₂-O-CO in which R₂ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted by one or a number of substituents, which are identical or different, chosen from the halogen atoms and the hydroxyl radical, alkoxy radical containing 1 to 4 carbon atoms, dialkylamino radical, each alkyl part of which contains 1 to 4 carbon atoms, piperidino radical, morpholino radical, 1-piperazinyl radical (optionally substituted in the 4-position by an alkyl radical containing 1 to 4 carbon atoms or by a phenylalkyl radical, the alkyl part of which contains 1 to 4 carbon atoms), cycloalkyl radical containing 3 to 6 carbon atoms, cycloalkenyl radical containing 4 to 6 carbon atoms, phenyl radical, cyano radical, nitro radical, carboxyl radical or alkoxycarbonyl radical, the alkyl part of which contains 1 to 4 carbon atoms,
- or a phenyl radical, optionally substituted by one or a number of radicals, which are identical or different, chosen from the alkyl radicals containing 1 to 4 carbon atoms or the alkoxy radicals containing 1 to 4 carbon atoms,
- or a saturated or unsaturated nitrogencontaining heterocyclyl radical containing 5 or 6 members, optionally substituted by one or a number of alkyl radicals containing 1 to 4 carbon atoms,
it being understood that the cycloalkyl, cycloalkenyl or bicycloalkyl radicals may optionally be substituted by one or a number of alkyl radicals containing 1 to 4 carbon atoms, and
Het represents an aromatic heterocyclic radical having 5 members and containing one or a number of atoms, which are identical or different, chosen from the nitrogen, oxygen or sulphur atoms, such as thiophene, thiazole, furan, pyrrole, imidazole, isoxazole or pyrazole, optionally substituted by one or a number of substituents, which are identical or different, chosen from the halogen atoms (fluorine, chlorine) and the alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, each alkyl part of which contains 1 to 4 carbon atoms, acylamino, the acyl part of which contains 1 to 4 carbon atoms, alkoxycarbonylamino, containing 1 to 4 carbon atoms, acyl, containing 1 to 4 carbon atoms, arylcarbonyl, the aryl part of which contains 6 to 10 carbon atoms, cyano, nitro, hydroxyl, carboxyl, carbamoyl, alkylcarbamoyl, the alkyl part of which contains 1 to 4 carbon atoms, dialkylcarbamoyl, each alkyl part of which contains 1 to 4 carbon atoms, or alkoxycarbonyl, the alkoxy part of which contains 1 to 4 carbon atoms, radicals, with the exception of Het representing a 2-thienyl or 2-furyl radical when R₁ represents a benzoyl radical or when R₂ represents an alkyl radical containing 1 to 6 carbon atoms.

28. New derivatives according to claim 26 for which R represents a hydrogen atom or an acetyl radical, R₁ represents a benzoyl radical or a radical R₂-O-CO- in which R₂ represents a t-butyl radical and Het represents a 3-thienyl or 3-furyl radical.

29. Pharmaceutical composition, characterized in that it contains at least one product according to claims 26, 27 or 28 in combination with one or more pharmaceutically acceptable products whether they are inert or physiologically active.
